# FASCICULE DE BREVET EUROPEEN

(11) **EP 3 821 239 B1**
(45) Date de publication et mention de la délivrance du brevet: **10.04.2024**
(21) Numéro de dépôt: 19736381.5
(22) Date de dépôt: 08.07.2019
(51) Int. Cl.: G06Q 10/04, E03B 7/07, E03B 7/02, G01N 33/18, G06Q 50/06

(54) **PLACEMENT AMELIORÉ DE CAPTEURS DE PARAMETRES PHYSICO-CHIMIQUES DANS UN FLUIDE**
VERBESSERTE PLATZIERUNG PHYSIKOCHEMISCHER PARAMETERSENSOREN IN EINER FLÜSSIGKEIT
IMPROVED PLACEMENT OF PHYSICO-CHEMICAL PARAMETER SENSORS IN A FLUID

(30) Priorité: 09.07.2018 US 201862695370 P; 10.10.2018 FR 1859402
(43) Date de publication de la demande: 19.05.2021
(73) Titulaire: Suez International, 92040 Paris La Défense Cedex (FR)
(72) Inventeur: CUSSONNEAU, Guillaume, 75010 Paris (FR); FAY, Gilles, 75019 Paris (FR); DO QUANG, Zdravka, 78870 Bailly (FR); LIER, Jean-Marc, 33600 Pessac (FR); RENARD, Jean-François, 91120 Palaiseau (FR); CHAZERAIN, Aurélie, 75009 Paris (FR)
(74) Mandataire: Lavoix
(86) Numéro de dépôt international: PCT/EP2019/068254
(87) Numéro de publication internationale: WO 2020/011711

(56) Documents cités:
- EP-A1- 3 112 960
- WO-A1-2018/001627
- US-B1- 9 964 468
- N. CHEIFETZ ET AL: "Un algorithme glouton pour le positionnement de capteurs qualité sur un grand réseau de distribution d'eau", TSM. TECHNIQUES SCIENCES METHODES, GENIE URBAIN GENIE RURAL, no. 11, 1 novembre 2017 (2017-11-01), pages 55-63, XP055625307, ISSN: 0299-7258, DOI: 10.1051/tsm/201711055

## Description

### DOMAINE DE L'INVENTION

La présente invention concerne le domaine des réseaux de distribution de fluides, tels que l'eau ou le gaz. Plus particulièrement, la présente invention concerne la détermination de positions optimisées de capteurs permettant des mesures des données physico-chimiques du fluide.

### ETAT DE L'ART PRECEDENT

Les réseaux de distribution d'eau peuvent être affectés par de nombreuses anomalies affectant la qualité de l'eau. Par exemple, l'eau peut être contaminée par différents polluants introduits volontairement (acte de malveillance) ou involontairement par retour d'eau ou erreur de connexion, par des particules de matières se déposant régulièrement dans les conduites et soudainement remises en suspension, par la réaction avec les matériaux constitutifs des réseaux, ou par des bactéries lors de phénomènes de recroissances bactériennes. Afin de prévenir les effets délétères pouvant être causés par la contamination, ou de manière plus générale par la diminution de la qualité de l'eau pouvant provoquer l'inconfort des usagers (changement de couleur, de goût, d'odeur ...), il convient de détecter et caractériser, de manière aussi rapide et précise que possible, tout événement affectant la qualité de l'eau. Des problèmes altérant la qualité de l'eau peuvent également survenir dans des systèmes, étendues ou cours d'eau naturels tels que des lacs, étangs ou rivières ou ensemble d'étendues et de cours d'eaux naturels. Ces systèmes peuvent être affectés par exemple par des pollutions accidentelles, ou la croissance anormale d'algues, dégradant significativement leur état et empêchant leur usage (potabilisation, eaux de baignade...).

Les réseaux de distribution d'eau peuvent également voir survenir d'autres anomalies comme les fuites, les pressions trop élevées ou trop basses, les vannes mal positionnées... D'autres réseaux de distribution de fluide, tels que des réseaux de distribution de gaz, ou des réseaux d'oléoducs, peuvent être affectés par des anomalies, par exemple des surpressions ou des fuites, nécessitant des actions correctrices.

L'utilisation de capteurs connectés a permis d'accroître considérablement l'efficacité de la détection d'anomalies dans des réseaux de distribution de fluide. De tels capteurs permettent de mesurer un ou plusieurs paramètres physico-chimiques (pression, vitesse, turbidité, pH...) du fluide en un point du réseau, et d'analyser localement ces valeurs de paramètres ou les transmettre à un serveur pour analyse. Ainsi, des anomalies ou pollutions d'un réseau de distribution de fluide peuvent être détectées automatiquement, et les actions correctives ou préventives peuvent être mises en place immédiatement. La demande de brevet français n° 1763286, déposée par la demanderesse de la présente demande, décrit un dispositif de détection d'anomalies dans un continuum d'eau à partir de mesures de paramètres physico-chimiques dans le continuum d'eau.

Des anomalies dans un réseau de distribution de fluide peuvent apparaître en divers points du réseau. Par exemple, une pollution dans un réseau de distribution d'eau peut apparaître dans un réservoir en entrée du réseau, mais également en un autre point du réseau, par exemple un équipement industriel dans lequel un retour d'eau serait déclenché. Une pollution ainsi créée ne peut être, par nature, détectée que lorsqu'une variation de paramètres anormale a pu être détectée par au moins un capteur, c'est-à-dire lorsque la pollution est arrivée à ce capteur. Une pollution non détectée est extrêmement problématique, en particulier pour des réseaux de distribution d'eau potable, pour lesquels elle peut affecter la santé des usagers du réseau. Elle peut aussi occasionner une gêne sans affecter la santé des usagers et nécessite généralement des actions curatives et dommageables pour la continuité du service.

La détection des anomalies peut être améliorée en augmentant le nombre de capteurs dans le réseau de distribution d'eau. Cependant, ces capteurs sont coûteux à l'achat et à l'usage, notamment du fait de leur maintenance. Il convient donc de permettre une détection d'anomalie aussi rapide que possible, pour la plus grande partie possible d'un réseau de distribution d'eau, avec un nombre de capteurs aussi faible que possible. Une disposition adéquate des capteurs est donc indispensable, pour parvenir à une détection satisfaisante d'anomalies avec un nombre de capteurs limités.

Le problème du placement optimal d'un nombre donné de capteurs dans un réseau de distribution d'eau est un problème combinatoire extrêmement complexe. Il est dans la pratique impossible de tester l'ensemble des solutions sur un réseau de distribution d'eau de taille standard (par exemple à l'échelle d'une ville ou d'une région), car un test exhaustif de toutes les combinaisons possibles de positions de capteurs nécessite des capacités de calcul trop importantes pour être mis en oeuvre en pratique.

Cheifetz, N., Sandraz, A. C., Feliers, C., Gilbert, D., Piller, O., & Heim, V. (2017). Un algorithme glouton pour le positionnement de capteurs qualité sur un grand réseau de distribution d'eau. Techniques Sciences Méthodes, (11), 55-63. proposent de placer des capteurs dans un réseau de distribution d'eau à l'aide d'un algorithme dit « glouton », c'est-à-dire en plaçant, de manière itérative, un nombre de capteurs donné un par un, à une position considérée comme optimale en combinaison avec les capteurs déjà placés. S'il permet d'obtenir un placement de capteurs assez satisfaisant à l'aide de capacités de calcul limitées, cet algorithme ne permet en général pas d'obtenir un placement optimal des capteurs. En effet, une fois un capteur placé, sa position ne peut plus être mise en cause, alors même que cette position, considérée comme optimale à partir des positions des capteurs précédemment placés, ne le serait plus avec un nombre de capteurs plus important. Des autres méthodes pour placer des capteurs sont décrites dans US9964468, WO2018001627 et EP3112960.

Il y a donc besoin d'une méthode permettant un placement optimal d'un nombre de capteurs donné dans un réseau de distribution d'eau, afin de détecter une anomalie dans le réseau de distribution d'eau dès que possible, quelle que soit la source de l'anomalie.

Il y a, de manière plus générale, besoin d'une méthode permettant un placement optimal d'un nombre de capteurs donné dans un réseau de distribution de fluide, afin d'optimiser la détection d'anomalies dans le fluide.

### RESUME DE L'INVENTION

L'invention atteint ce but en simulant des scénarios introduisant, pour certains au moins, des anomalies dans le fluide ; en déterminant des ensembles candidats de positions capteurs ; en déterminant les valeurs simulées des paramètres du fluide en chaque position et dans chaque scénario, et la détection d'anomalie associée ; en attribuant un score à chaque ensemble candidat de position capteurs, représentant l'efficacité avec laquelle les capteurs, placés aux positions de chaque ensemble candidat, ont pu détecter les anomalies. Enfin, les ensembles candidats peuvent être modifiés à l'aide d'algorithmes dits génétiques jusqu'à ce qu'un critère d'arrêt soit validé. Les algorithmes génétiques peuvent par exemple consister à croiser ou muter des ensembles candidats.

A cet effet, l'invention décrit une méthode de placement d'une pluralité de capteurs d'un ou plusieurs paramètres physico-chimiques d'un fluide telle que définie dans la revendication 1 du jeu de revendication attaché.

Par « pluralité d'ensembles » on entend, dans la présente description, un ensemble de combinaisons de positions dans le réseau d'un nombre de capteurs donné. L'optimisation vise ainsi à déterminer pour un nombre de capteurs donné la meilleure combinaison pour obtenir le score de performance le plus élevé. On définit une pluralité de combinaisons à tester et qu'on fait évoluer à chaque itération grâce à l'étape de modification de la pluralité d'ensembles.

Un critère d'arrêt est une combinaison de règles basées sur les performances des populations évaluées dans le processus d'optimisation à chaque itération et de règles complémentaires indépendantes : - règle basée sur les performances mesurées à chaque itération : un certain nombre d'itérations successives ont été effectuées sans que les performances des meilleurs ensembles, par exemple des 5% de la population ayant les meilleures performances n'évoluent de plus d'un certain pourcentage. On atteint un plateau - règle indépendante des performances : un nombre d'itérations maximal fixé au préalable est atteint. Ce nombre d'itérations maximal dépend de la complexité du problème (il augmente quand le système à optimiser est plus complexe et nécessité plus d'itérations).

« Le score le plus favorable » est la valeur de la fonction objectif calculée pour une combinaison de position de capteurs qui fournit le résultat remplissant le mieux les objectifs assignés au placement de capteurs. Selon les cas, le score le plus favorable est une valeur supérieure à une autre valeur de la fonction objectif pour une combinaison de position de capteurs moins favorable. Par exemple, si le score correspond à un nombre de noeuds protégés par la combinaison de capteurs, le score le plus favorable sera le score le plus élevé, correspondant à un plus grand nombre de capteurs protégés. Si, au contraire, le score correspond à un nombre de noeud pour lesquels une anomalie n'a pas été détectée, ou a été détectée trop tard, le score le plus favorable correspond à la valeur la plus faible, et donc à un nombre de noeuds pour lesquels l'anomalie n'a pas été détectée aussi faible que possible. Le score peut être calculé à partir d'un ou plusieurs éléments : capacité de détection d'anomalies, nombre d'usagers pour lesquels les anomalies sont détectées à temps, consommation équivalente pour laquelle les anomalies sont détectées à temps, rapidité des détections d'anomalies, détections d'un groupe d'anomalies ayant un fort impact sur les usagers, coût de déploiement des capteurs, etc...

Les algorithmes de type génétique consistent à modifier entre deux itérations, les ensembles candidats, en utilisant une ou plusieurs opérations choisies parmi :
- la conservation : on conserve une partie des candidats ayant les scores les plus favorables, et on supprime les autres ;
- le croisement : on combine les positions des capteurs d'au moins deux ensembles. Les ensembles entre lesquels on effectue un croisement peuvent être des ensembles préalablement sélectionnés ;
- la mutation : on modifie une ou plusieurs positions de capteurs d'un ensemble, par exemple de manière aléatoire.

La modification des ensembles candidats de positions capteurs, à l'aide d'algorithmes de type génétique permet dans tous les cas d'aboutir à un placement de capteurs proche du placement optimal.

La méthode présente une complexité algorithmique relativement faible(par exemple, la méthode est beaucoup moins gourmande que d'autres méthodes d'optimisation telles que la méthode « brute force » consistant à tester toutes les options), et peut s'exécuter sur des capacités de calcul communes, telles que des ordinateurs individuels ou serveurs, avec des temps d'exécutions différents mais restant raisonnables. Une méthode selon l'invention, appliquée à un réseau de complexité moyenne peut ainsi s'exécuter en quelques minutes à quelques heures sur un ordinateur personnel, alors qu'une méthode « brute force » sera beaucoup plus longue à exécuter voir, en pratique, inexécutable sur un tel ordinateur.

La méthode est applicable à tout type de réseau de distribution de fluide.

La méthode permet de placer des capteurs dans un réseau, pour détecter des anomalies de manière optimale à moindre coût.

La méthode permet d'assurer un niveau de sécurité des usagers du réseau avec un nombre limité de capteurs.

Dans des exemples de réalisation, le réseau est modélisé sous forme d'un graphe ; chaque noeud ou arc du graphe est identifié par un identifiant unique ; la position d'un capteur est définie par un identifiant d'un noeud ou arc du graphe.

Cette représentation permet une identification simple et efficace des positions des capteurs.

Dans des exemples de réalisation, au moins un des ensembles de paramètres d'entrée comprend l'introduction d'une anomalie dans au moins un point du réseau à au moins un pas de temps.

Avantageusement ceci permet un placement de capteurs optimisant la détection d'anomalie dans le réseau.

Dans des exemples de réalisation, le score de performance est calculé en fonction de la capacité des capteurs placés dans l'ensemble candidat de positions à détecter l'au moins une anomalie en déterminant un pas de temps d'arrivée de l'au moins une anomalie en un ensemble de points du réseau, et au moins une fonction objectif choisie parmi : un nombre de points de l'ensemble, pour lesquels l'anomalie est détectée avant son arrivée; un nombre de points de l'ensemble, pour lesquels l'anomalie est détectée avant son arrivée, pondérés par un nombre d'usagers ou une consommation par point ; un nombre de points pour lesquels l'anomalie n'a pas été détectée ; un nombre de points pour lesquels l'anomalie n'a pas été détectée, pondérés par un nombre d'usagers ou une consommation par point.

Ceci permet de placer les capteurs de manière optimale, afin d'éviter qu'une anomalie ayant un fort impact (par exemple au niveau d'un réservoir) ne soit pas détectée avant d'impacter des usagers du réseau.

Dans des exemples de réalisation, le score de performance est calculé à partir d'au moins une caractéristique, choisie parmi une capacité des capteurs d'un ensemble candidat à évaluer un indicateur de qualité du fluide dans le réseau, et un coût de déploiement des capteurs.

Ceci permet d'optimiser le placement de capteurs, de manière combinée, pour la détection d'anomalies, et d'autres objectifs tels que la traçabilité de la qualité du fluide dans l'ensemble des sous-parties du réseau, la protection des sites sensibles ou la limitation du coût de déploiement.

Dans des exemples de réalisation, au moins un capteur a une position prédéfinie.

Ceci permet de prendre en compte la présence de capteurs préexistants, et d'optimiser le placement de capteurs additionnels par rapport aux capteurs préexistants.

Dans des exemples de réalisation, les points auxquels les capteurs sont placés sont restreints à un sous-ensemble des points du réseau.

Ceci permet de prendre en compte que certains points du réseau ne permettent pas le placement de capteurs, et d'optimiser le placement des capteurs par rapport aux points de placement possibles. Des visites terrain peuvent permettre de sélectionner des sites, sur la base de connaissances préalables des infrastructures du réseau, et d'en invalider certains présentant des contraintes trop importantes ou un coût de réalisation prohibitif.

Dans des exemples de réalisation, la définition de la pluralité d'ensembles candidats de positions de la pluralité de capteurs comprend la définition de positions à des points d'intérêt du réseau.

Ceci permet de placer, dès la première itération, des capteurs en des points sensibles, tels que des sites industriels, des zones d'interconnexion avec achat d'eau en gros ou des sorties de réservoirs. Ceci permet ainsi de disposer, dès la première itération, d'un placement performant des capteurs. La méthode peut donc converger plus rapidement vers une solution proche d'un optimum, et nécessite une moins grande complexité de calcul pour s'exécuter.

Dans des exemples de réalisation, la définition de la pluralité d'ensembles candidats de positions de la pluralité de capteurs comprend la définition de positions à des noeuds connectés à un nombre d'arcs supérieur ou égal à un seuil prédéfini.

Ceci permet de ne placer des capteurs qu'à des noeuds connectés à un nombre d'arcs supérieur au seuil à la première itération (par exemple des noeuds connectés à au moins 2, 3 ou 4 arcs). Ces noeuds sont, statistiquement, plus susceptibles de détecter l'arrivée d'une anomalie ou de protéger un plus grand nombre de noeuds à l'aval. Ceci permet ainsi de disposer, dès la première itération, d'un placement performant des capteurs. La méthode est donc susceptible de converger vers une solution proche de l'optimum, et nécessite une moins grande complexité de calcul pour s'exécuter.

Dans des exemples de réalisation, le critère d'arrêt comprend une ou plusieurs conditions choisies parmi : un nombre d'itérations maximal ; une comparaison du score le plus favorable parmi l'itération courante et au moins une itération précédente, et la validation du critère d'arrêt si une différence entre le score le plus favorable à l'itération courante et à l'au moins une itération précédente est inférieure ou égale à un seuil prédéfini.

Un critère d'arrêt basé sur un nombre d'itération maximal permet de borner le temps d'exécution, et les ressources de calcul requises par la méthode.

Un critère d'arrêt basé sur une comparaison entre les scores les plus favorables entre l'itération courante et les itérations précédentes permet d'arrêter la méthode lorsque le gain d'optimisation du placement des capteurs entre au moins deux itérations successives devient marginal. Ceci permet d'exécuter un nombre d'itération plus faible une fois le score parvenu sur un plateau.

Dans des exemples de réalisation, la suppression d'au moins un ensemble candidat de positions n'ayant pas le score le plus favorable comprend la suppression de tous les ensembles candidats sauf un nombre prédéfini, ou un ratio prédéfini des ensembles candidats ayant le score le plus favorable ; l'ajout d'au moins un ensemble candidat des positions de capteurs défini par une combinaison des positions d'au moins deux d'ensembles candidats, et l'ajout d'au moins un ensemble candidat des positions de capteurs défini par une modification d'une position d'un capteur dans un ensemble candidat ajoutent un nombre d'ensembles candidats égal au nombre d'ensembles candidats supprimés.

Ceci permet de conserver un nombre d'ensemble candidats constant entre les itérations, et de conserver un nombre donné d'ensembles candidats ayant les scores les plus favorables entre deux itérations. Ainsi, le score le plus favorable parmi les ensembles à une itération est nécessairement au moins égal au score le plus favorable à l'itération précédente. De plus, ceci permet de conserver un vivier d'ensembles candidats ayant de bonnes performances, qui seront plus à même, par le biais de mutations ou de croisements, de générer de nouveaux candidats ayant de bonnes performances. Cela permet à la méthode de converger plus rapidement.

Dans des exemples de réalisation, la méthode comprend la définition, la modification de manière itérative, et la sélection d'ensembles candidats de capteurs pour une pluralité de nombres prédéfinis de capteurs respectivement.

Ceci permet de comparer les performances de différents nombres de capteurs.

Dans des exemples de réalisation, l'obtention d'une pluralité d'ensembles candidats de positions de la pluralité de capteurs pour un nombre entier (n) de capteurs est basée sur l'ensemble candidat de position ayant le score le plus favorable pour un nombre entier (n-m) de capteurs, avec 1 ≤ m < n.

Ceci permet de se baser sur le placement le plus performant pour un nombre (n-m) de capteurs pour le placement initial de (n) capteurs, ce placement le plus performant de (n-m) capteurs fournissant a priori une bonne base pour un placement performant de (n) capteurs. m est ici un entier compris entre 1 et n.

Dans des exemples de réalisation, la méthode comprend la sélection d'un des ensembles candidats ayant le score le plus favorable pour la pluralité de nombres prédéfinis de capteurs, en fonctions des scores et coûts de déploiement de chacun desdits ensembles candidats.

Ceci permet de sélectionner le nombre de capteurs présentant le meilleur compromis entre coût de déploiement et efficacité de détection d'anomalies.

L'invention décrit également un produit programme d'ordinateur comprenant des instructions de code de programme qui, lorsque le programme est exécuté par un ordinateur, conduisent celui-ci à mettre en oeuvre la méthode selon l'un des modes de réalisation de l'invention..

L'invention décrit également un dispositif apte à déterminer un ensemble de positions, dans un réseau de transport d'un fluide, d'une pluralité de capteurs d'un ou plusieurs paramètres physico-chimiques du fluide, ledit dispositif comprenant un processeur configuré pour exécuter une méthode de détermination d'un ensemble de positions, dans un réseau de transport d'un fluide, d'une pluralité de capteurs d'un ou plusieurs paramètres physico-chimiques du fluide selon l'invention.

Dans des exemples de réalisation, le dispositif peut être un capteur apte à calculer sa position optimale et la montrer à travers des moyens de visualisation à un utilisateur qui positionnera le capteur dans la position optimale, en prenant en compte l'ensemble des capteurs déjà déployés ou en cours de déploiement.

### LISTE DES FIGURES

D'autres caractéristiques apparaîtront à la lecture de la description détaillée donnée à titre d'exemple et non limitative qui suit faite au regard de dessins annexés qui représentent :
- la figure 1 représente un exemple de sonde destinée à détecter des anomalies dans réseau de distribution d'eau, pouvant être placée par une méthode selon l'invention ;
- la figure 2 représente un diagramme de flux d'une méthode de placement de capteurs selon l'invention ;
- la figure 3 représente un exemple de réseau de distribution d'eau auquel l'invention peut être appliquée ;
- la figure 4 représente un exemple de détection d'anomalie dans un réseau de distribution de fluide selon un ensemble de modes de réalisation de l'invention ;
- la figure 5 représente un exemple de définition de contraintes d'optimisation selon un ensemble de modes de mise en oeuvre de l'invention ;
- la figure 6 représente un exemple d'histogramme de différents scénarios de tests pour le placement de capteurs avec leurs pourcentages de noeud d'un réseau de distribution d'eau impactés par chaque pollution ;
- les figures 7a, 7b, 7c et 7d représentent quatre exemples de placement de 2, 5, 8 et 11 capteurs respectivement dans un réseau de distribution d'eau par une méthode de placement de capteurs dans un mode de réalisation de l'invention visant uniquement la détection de pollution ;
- la figure 8 représente l'évolution d'un pourcentage de détection d'anomalies par des capteurs dans un réseau de distribution d'eau placés par une méthode selon l'invention, en fonction du nombre de capteurs ;
- la figure 9 représente un exemple de répartition des débits dans un réseau de distribution d'eau ;
- la figure 10 représente un exemple de suivi de la qualité de l'eau par des capteurs situés sur des noeuds en aval d'un réseau de distribution d'eau ;
- les figures 11a, 11b, 11c et 11d représentent quatre exemples de placement de 2, 5, 8 et 11 capteurs respectivement dans un réseau de distribution d'eau par une méthode de placement de capteurs dans un mode de réalisation de l'invention visant à la fois la détection de pollution et le suivi de la qualité de l'eau à l'aval ;
- la figure 12 représente l'évolution d'un score combiné de détection de pollution et de suivi de la qualité de l'eau en fonction d'un nombre de capteurs placés par une méthode selon l'invention.

### DESCRIPTION DETAILLEE

Dans la suite de la description le procédé selon l'invention est principalement illustré par des exemples relatifs au placement optimisé de capteurs pour la détection de pollution et le suivi de qualité de l'eau dans un réseau de distribution d'eau potable. Cependant, l'invention n'est pas restreinte à ces exemples, et peut être appliquée au placement de capteurs pour la détection d'autres anomalies dans un réseau d'eau, telles que des fuites, ainsi qu'à d'autres objectifs. L'invention peut également être appliquée au placement optimisé de capteurs dans d'autres réseaux de distribution de fluide, tels que des réseaux de distribution de gaz, ou des réseaux d'oléoducs.

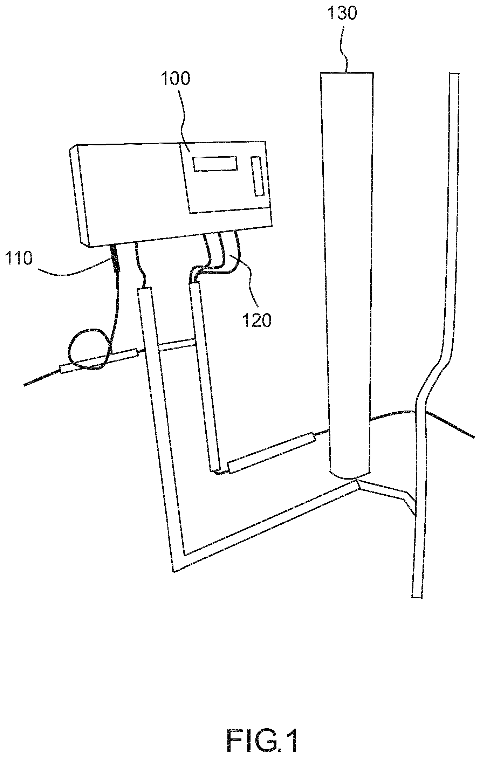

La figure 1 représente un exemple de sonde destinée à détecter des anomalies dans réseau de distribution d'eau, pouvant être placée par une méthode selon l'invention.

La sonde 100 est destinée à détecter des anomalies dans un réseau de distribution d'eau. La sonde peut être placée à n'importe quel emplacement d'un réseau de distribution d'eau, par exemple en sortie d'une usine de production d'eau potable, en sortie de réservoir, à un point de consommation, où en tout autre point. La demanderesse a déposé une demande de brevet français n° 1763286 portant sur une sonde telle que la sonde 100, permettant de déterminer les anomalies survenant dans un continuum d'eau.

La sonde 100 est connectée à des canalisations 130 du réseau de distribution d'eau, par exemple par un ensemble d e câbles de capteurs 120 ou directement connectée sur le réseau (insertion), et est alimentée en électricité 110 ou sur batterie.

La sonde 100 peut comprendre un ou plusieurs capteurs de grandeurs physiques du réseau de distribution d'eau. Par exemple, la sonde 100 peut comprendre un ou plusieurs capteurs choisis parmi des capteurs de concentration en chlore, température, TOC (de l'anglais Total Organic Carbon, signifiant Carbone Organique Total), UV 254 (absorbance de l'eau pour une lumière ultraviolette de longueur d'onde 254 nm), conductivité, pH, couleur, turbidité, nombre de particules, nombre de bactéries, oxygène dissous, chlorophylle a ou tout capteur d'une grandeur physique pouvant caractériser l'eau.

La sonde 100 permet ainsi, dans un ensemble de modes de réalisation de l'invention, d'effectuer des mesures d'un ensemble de paramètres représentatifs de la qualité de l'eau en un point du réseau de distribution d'eau.

Dans un ensemble de modes de réalisation de l'invention, la sonde 100 comprend des moyens des communications afin de transmettre les mesures des capteurs embarqués. Par exemple, la sonde peut comprendre une connexion filaire ou radio vers un serveur afin d'envoyer les mesures à un serveur configuré pour détecter des anomalies dans le réseau de distribution d'eau. La sonde 100 peut ainsi être couplée à un capteur intelligent de consommation d'eau envoyant des données de consommation en télé-relève, en envoyant, de manière combinée, des données de consommation d'eau et mesures des capteurs.

Dans un ensemble de modes de réalisation de l'invention, la sonde comprend un processeur configuré pour détecter et caractériser, à partir des mesures des capteurs, des anomalies dans le réseau de distribution d'eau.

Des exemples de détection et de caractérisation d'anomalies par un processeur seront fournies, par exemple dans la demande de brevet français n° 1763286.

Bien que la sonde 100 représente un exemple de sonde dans un réseau de distribution d'eau, de telles sondes peuvent également être déployées dans de l'eau en milieu naturel, par exemple dans un lac, étang, rivière ou dans tout autre système aquatique, et ceci éventuellement à différentes profondeurs.

Une sonde telle que la sonde 100 permet de détecter des anomalies telles que des pollutions dans un réseau de distribution d'eau. Cependant, ces anomalies ne peuvent être détectées qu'à partir des mesures effectuées par la sonde. Une pollution générée en amont de la sonde 100 peut donc affecter des usagers, sans avoir été détectée. Il convient donc, pour détecter des pollutions dans un réseau de distribution d'eau suffisamment tôt pour protéger les usagers, de disposer plusieurs sondes dans le réseau. Cependant, une sonde telle que la sonde 100 présente un coût de fabrication, d'utilisation et de maintenance non négligeable.

Un objectif de l'invention est de réaliser un placement optimal d'un nombre limité de sondes dans un réseau de distribution d'eau, afin d'obtenir une détection d'anomalies aussi performante que possible, et ainsi une bonne protection des usagers, tout en conservant un coût économique raisonnable. De manière plus générale, un objectif de l'invention est de placer, dans un réseau de distribution de fluides, un nombre limité de capteurs permettant la détection d'anomalies dans le réseau, de manière optimale afin d'obtenir la détection d'anomalies la plus performante avec un coût d'exploitation limité.

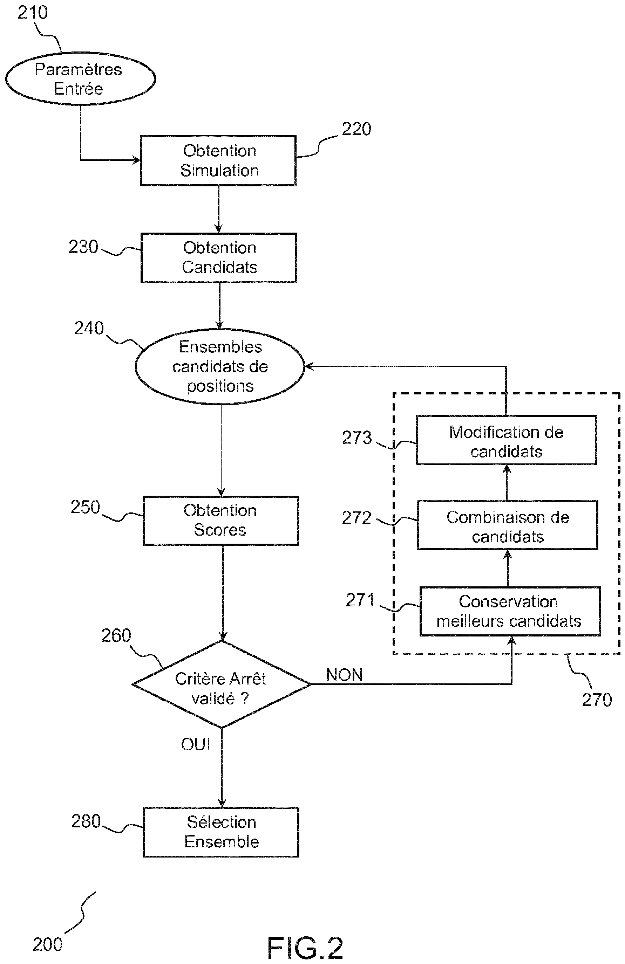

La figure 2 représente un diagramme de flux d'une méthode de placement de capteurs selon l'invention.

La méthode 200 est une méthode de placement d'une pluralité de capteurs d'un ou plusieurs paramètres physico-chimiques d'un fluide comprenant l'exécution des étapes d'une méthode mise en oeuvre par ordinateur de détermination d'un ensemble de positions, dans un réseau de transport d'un fluide, d'une pluralité capteurs d'un ou plusieurs paramètres physico-chimiques du fluide. Le réseau de distribution de fluide peut être par exemple un réseau de distribution d'eau, de gaz, ou un réseau d'oléoducs.

La méthode 200 se base sur au moins un ensemble de paramètres d'entrée 210. Les paramètres d'entrée permettent de définir un scénario de simulation, en définissant un état initial du réseau, et des paramètres venant modifier l'état du réseau. Le fonctionnement du réseau peut ainsi être simulé pendant un ensemble de pas de temps. Les paramètres modifiant l'état du réseau peuvent correspondre au fonctionnement habituel du réseau (par exemple, un profil de consommation d'eau en un point du réseau), ou l'introduction d'une anomalie à un pas de temps donné. L'introduction de l'anomalie peut se faire de différentes manières. Par exemple, l'anomalie peut être introduite à un point prédéterminé correspondant à un point d'intérêt du réseau (par exemple, l'introduction d'une pollution en sortie d'une usine, ou au niveau d'un poteau incendie). Les anomalies peuvent également être introduites, de manière aléatoire, en différents points du réseau. Les anomalies peuvent correspondre à des éléments de gravité variable, tels que des fuites, des surpressions, ou des pollutions accidentelles ou volontaires (sabotage) du réseau.

La méthode 200 comprend l'obtention 220, pour l'au moins un ensemble de paramètres d'entrée 210, de valeurs simulées de grandeurs physico-chimiques en un ensemble de points du réseau pour un ensemble de pas de temps. Ainsi, le fonctionnement du réseau peut être simulé aux différents pas de temps, différentes grandeurs (température, pH, vitesse, turbidité...) étant simulées en différents points du réseau pour l'ensemble de pas de temps. La simulation peut être faite à chaque exécution de la méthode. Elle peut également être exécutée une seule fois, par exemple à l'initialisation de la méthode ou lors de la première itération, ou avoir été pré-calculée. Dans ce cas, il suffit de récupérer les valeurs simulées pour obtenir le comportement du réseau dans un scénario de simulation.

La méthode 200 comprend ensuite l'obtention 230 d'une pluralité d'ensemble candidats 240 de positions de la pluralité de capteurs. En effet, la méthode 200 vise à déterminer une combinaison optimale de positions d'un ensemble de capteurs. Comme il sera expliqué ci-après, la méthode teste différents ensembles candidats de positions, puis les modifie de manière itérative afin d'identifier des combinaisons plus performantes, itération après itération.

Le réseau, et les positions des capteurs, peuvent être représentés de différentes manières. Par exemple, le réseau peut être modélisé sous forme d'un graphe formé de noeuds et d'arcs. Les noeuds peuvent correspondre à des lieux de production ou de consommation du fluide (i.e réservoir, regroupement de consommateurs, usine...), et les arcs aux différentes infrastructures permettant de faire transiter le fluide entre les noeuds, par exemple des canalisations. Chaque noeud ou arc peut être identifié par un identifiant unique, la position d'un capteur peut alors être définie par l'identifiant du noeud ou arc auquel il est situé.

L'obtention 230 des ensembles de positions de capteurs permet d'avoir des ensembles de positions initiales. Pour les ensembles de capteurs. Ces positions initiales peuvent être obtenues de plusieurs manières.

Par exemple, au moins un capteur peut avoir une position prédéfinie. Il peut par exemple s'agir de capteurs déjà disposés dans le réseau, dont la position reste fixe. Ainsi, le positionnement de capteurs additionnels peut être optimisé en tenant en compte les capteurs déjà présents.

Les positionnements des capteurs peuvent aussi être restreints à un sous-ensemble de points du réseau, par exemple certains noeuds et/ou arcs. Ceci permet par exemple d'éviter de placer des capteurs à des emplacements inaccessibles, des emplacements pour lesquels le déploiement de capteurs serait trop coûteux, des emplacements ne disposant pas de l'espace nécessaire pour disposer un capteur, ou ne permettant pas un niveau de transmission suffisant, etc...

Parmi les points de placement possibles, les capteurs peuvent par exemple être initialement disposés de manière aléatoire. Les positions peuvent également être définies à partir de points d'intérêts du réseau (par exemple, des usines, retenues d'eau, etc... ces points représentant de bons candidats pour un placement optimal de capteur.

Les capteurs peuvent également être placés uniquement dans un sous-ensemble de points correspondant à des bons candidats pour l'optimisation. Ceci permet d'obtenir de bonnes performances, tout en réduisant la complexité de la méthode. Les bons candidats peuvent être par exemple les points ayant à eux seuls un score de performance élevé selon l'objectif recherché.

Les capteurs peuvent également être placés à des points du réseau connectés à un nombre d'arcs important, par exemple, uniquement à des noeuds connectés à un nombre d'arcs supérieur ou égal à un seuil prédéfini.

Ces différents types de placements initiaux peuvent être combinés. D'autres manières d'obtenir un placement initial de capteur sont également envisageables. De manière générale, il est désirable que le placement initial des capteurs permette aux mesures issues des capteurs de détecter des phénomènes ayant lieu dans le réseau de manière aussi efficace que possible.

La méthode 200 comprend, de manière itérative, l'obtention de scores de performance pour les ensembles candidats de capteurs, et la modification des ensembles candidats à l'aide d'algorithmes de type génétique.

Les itérations comprennent ensuite l'obtention 250, pour chaque ensemble candidat de positions, d'un score de performance au moins à partir valeurs simulées de grandeurs physico-chimiques aux positions de la pluralité de capteurs. Le score de performance permet à partir des mesures capteurs, de déterminer à quel point un ensemble de positions de capteurs permet de remplir une mission donnée.

Dans un ensemble de modes de réalisations de l'invention, les valeurs simulées de grandeurs physico-chimiques sont directement obtenues pour les positions capteurs, et le score de performance calculé à partir de ces valeurs.

La méthode 200 peut également comprendre une étape intermédiaire d'obtention de prédictions de mesures des capteurs pour chaque ensemble candidat de positions parmi ladite pluralité pour chaque ensemble de paramètres d'entrée. Ceci permet de simuler, pour chaque scénario et chaque ensemble candidat, les mesures qui seront effectuées par les capteurs. Les mesures peuvent être considérées comme « parfaites », c'est-à-dire que le capteur mesure, à chaque pas de temps, exactement la valeur d'un paramètre à ce pas de temps à la position du capteur. Il est également possible de simuler les imperfections des capteurs, par exemple en utilisant un modèle de mesure capteur. Différents types de modèles peuvent être utilisés, représentant par exemple l'erreur de mesure à l'aide d'un bruit gaussien, la granularité temporelle des mesures à l'aide d'une fenêtre glissante, etc... Le placement des capteurs peut ainsi être optimisé à l'aide d'une modélisation plus réaliste de ce qui sera perçu par les capteurs.

L'obtention des prédictions de mesures capteur peut ainsi se faire en calculant les mesures, en sélectionnant les mesures parmi les valeurs simulées des paramètres physico-chimiques sur le réseau, ou en récupérant des prédictions de mesures pré-calculées.

Le score de performance peut ainsi être déterminé de différentes manières, en fonction de l'objectif suivi. Le score de performance peut être calculé à partir d'une fonction objectif représentant un objectif à optimiser à l'aide du placement adéquat des capteurs. Cette fonction objectif peut par exemple refléter la capacité des capteurs à détecter des pollutions avant qu'elles ne parviennent aux usagers ; la capacité des capteurs à détecter des anomalies dans le réseau ; la capacité des capteurs à évaluer un indice de qualité du fluide dans le réseau ; le coût de déploiement des capteurs ; une combinaison de plusieurs facteurs, choisis par exemple parmi ceux indiqués précédemment. De manière générale, le score de performance peut être défini de manière à permettre une efficacité maximale des capteurs à un coût de déploiement aussi faible que possible.

A l'issue de la première itération d'obtention 250 des scores de performances, la méthode 200 comprend une modification de la pluralité d'ensembles candidats des positions des capteurs. Cette modification est effectuée par le biais d'algorithmes dits génétiques, c'est-à-dire par le biais d'au moins une opération choisie parmi :
- la conservation 271 d'au moins un ensemble candidat de positions ayant un score le plus favorable. Cette opération dite de sélection consiste à conserver les meilleurs ensembles de candidats, c'est-à-dire ceux ayant obtenu le meilleur score. Ceci permet de s'assurer qu'à chaque itération le meilleur score parmi les ensembles candidats sera au moins aussi bon qu'à l'itération précédente. De plus, cela permet de conserver un vivier de très bons candidats, qui sera amélioré au fur et à mesure des itérations. A chaque itération, les candidats non conservés sont remplacés par de nouveaux, par exemple par les opérations suivantes ;
- l'ajout 272 d'au moins un ensemble candidat des positions de capteurs défini par une combinaison des positions d'au moins deux ensembles candidats. Cette opération dite de croisement consiste à combiner des positions issues de plusieurs ensembles candidats ;
- l'ajout 273 d'au moins un ensemble candidat des positions de capteurs défini par une modification d'une position d'un capteur dans un ensemble candidat. Cette opération dite de mutation consiste à modifier aléatoirement la position d'au moins un capteur dans un ensemble candidat.

Les croisements et mutations peuvent ainsi ajouter à l'ensemble un nombre de candidats égal au nombre de candidats non conservés, afin de conserver un nombre de candidats constant.

Les différentes opérations peuvent être combinées de différentes manières, générant un pourcentage de candidats pour chaque itération. Dans un ensemble de mode de réalisation de l'invention, on sélectionne les 5% de candidats ayant le meilleur score, on croise les 80% de candidats ayant un score intermédiaire, et on fait muter de manière aléatoire les 15% restants.

A partir de la 2^{e} itération de calcul des scores, c'est-à-dire après une première modification des ensembles au moins, la validation 260 d'un critère d'arrêt peut être vérifiée, à l'issue des calculs des scores. Cette validation peut être vérifiée à chaque itération, ou pour certaines itérations seulement (par exemple une itération sur 2, une itération sur 3, etc...) La validation du critère d'arrêt peut également être vérifiée, seulement à partir d'un nombre donné d'itérations (par exemple à partir de 10, 15, 20 itérations).

Le critère d'arrêt peut être basé sur une ou plusieurs conditions. Par exemple, le critère d'arrêt peut être basé sur un nombre d'itérations maximal. Le critère d'arrêt peut également être basé sur des différences entre le score le plus favorable à l'itération courante, et le score le plus favorable à au moins une itération précédente. Dans ce cas, le critère d'arrêt est validé lorsque, à l'issue d'une ou plusieurs itérations successives, le score le plus favorable n'évolue plus, ou seulement de manière marginale.

Le critère d'arrêt peut également être basé sur une combinaison de ces conditions. Par exemple, le critère d'arrêt peut être validé quand un nombre maximal d'itérations est atteint et/ou lorsque le score le plus favorable n'évolue plus à l'issue d'une ou plusieurs itérations.

Tant que le critère d'arrêt n'est pas validé 260, de nouvelles itérations de modification des ensemble candidats, et calculs de scores sont effectuées.

Lorsque le critère d'arrêt est validé 260, la méthode 200 comprend la sélection 290 de l'ensemble candidat de positions ayant le score le plus favorable.

La méthode 200 permet ainsi d'obtenir un ensemble de positions des capteurs optimal, pour réaliser une fonctionnalité désirée (détection d'anomalie, suivi de la traçabilité de la qualité du fluide...). La méthode 200 présente l'avantage de pouvoir s'effectuer dans un temps raisonnable avec des capacités de calcul courantes, tout en identifiant un résultat optimal, ou quasi-optimal.

Dans un ensemble de modes de réalisation de l'invention, la méthode 200 peut être effectuée pour différents nombres de capteurs, par exemple plusieurs nombres prédéfinis de capteurs.

Dans un ensemble de modes de réalisation de l'invention, la méthode est effectuée de manière indépendante pour les différents nombres de capteurs.

Dans un ensemble de modes de réalisation de l'invention, l'obtention 230 des ensembles candidats de positions initiaux est basée au moins en partie sur l'ensemble sélectionné pour un nombre de capteur inférieur. Par exemple, l'ensemble optimal pour un nombre de capteur égal à 12 peut servir de base pour un ensemble initial de capteurs candidats ayant 13 ou 14 capteurs. En effet, on peut supposer que cet ensemble optimal, auquel quelques capteurs seraient rajoutés, peut fournir un bon premier candidat pour un nombre de capteurs plus important.

Il est alors possible, à partir des meilleurs candidats pour différents nombres de capteurs, de sélectionner celui qui présente le meilleur rapport entre score de performance et coût de déploiement ou nombre de capteurs.

Une fois les positions des capteurs déterminées, leur placement peut se faire de différentes manières. L'invention porte ainsi également sur une méthode pour placer des capteurs à des positions optimisées. Dans un ensemble de modes de réalisation de l'invention, les capteurs sont disposés aux positions optimisées, par exemple par des opérateurs.

Dans un ensemble de modes de réalisation de l'invention, les capteurs ont la capacité de se placer eux-mêmes. C'est par exemple le cas de robot capteurs automatiques capables de se déplacer dans le réseau. De tels capteurs peuvent se localiser de différentes manières, par exemple à l'aide d'un récepteur GPS, à l'aide d'un positionnement par rapport à des émetteurs radio fixes (positionnement par rapport à des antennes 3G, Wi-Fi, etc... dont l'emplacement est connu). De tels robots peuvent aussi se positionner par rapport à une carte du réseau, si le robot est équipé d'une carte topographique du réseau, et est capable de repérer l'emplacement du réseau auquel il se trouve, par exemple à l'aide d'une caméra. Ainsi, un robot-capteur peut, de manière autonome, déterminer sa position optimale en tenant compte des capteurs déjà présents, et s'y placer automatiquement. Ceci présente l'avantage de ne pas nécessiter l'intervention d'un opérateur pour place les capteurs.

Un ensemble de robot-capteurs peut également opérer en réseau, en se plaçant automatiquement aux positions optimales les uns par rapport aux autres. De plus, les capteurs peuvent alors automatiquement, lorsque la situation dans le réseau évolue (par exemple si un nouveau capteur est ajouté, un capteur déplacé sur un autre réseau, un capteur est défaillant...), recalculer leurs positions optimales, et s'y déplacer, sans besoin de l'intervention d'un opérateur.

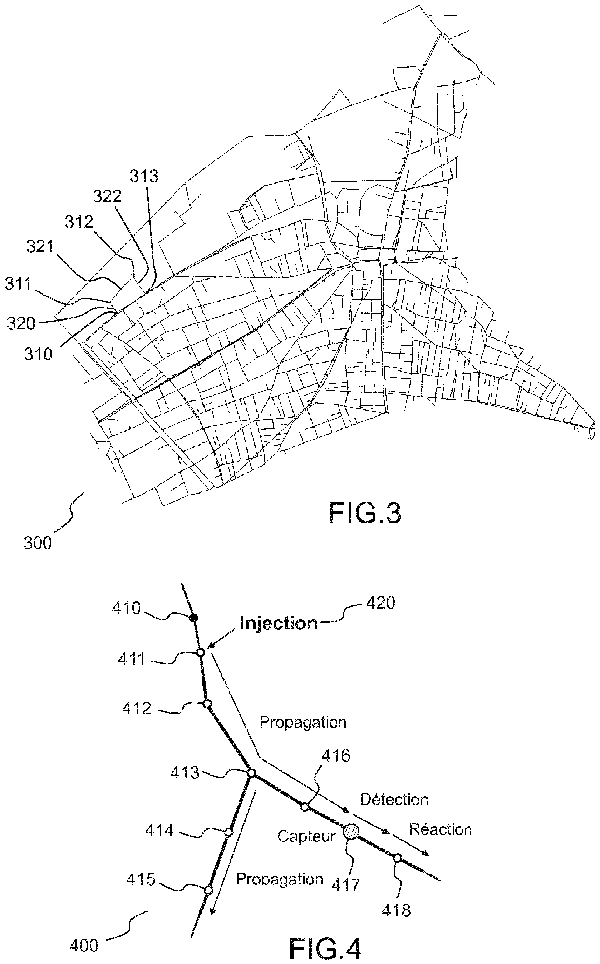

La figure 3 représente un exemple de réseau de distribution d'eau auquel l'invention peut être appliquée.

Le réseau 300 de distribution d'eau est un réseau à l'échelle d'une ville. Le secteur 300 est inclus dans un réseau de grande taille, comptant environ 25000 noeuds. Ce secteur est décrit par un modèle hydraulique, représenté en figure 3, composé de 3305 noeuds, par exemple les noeuds 310, 311, 312, 313 représentant les points de consommation, et 3639 arcs, par exemple les arcs 320, 321, 322. représentant les conduites. Les noeuds sont connectés à différents nombres de conduites. Par exemple, les noeuds 311 et 312 sont connectés à deux conduites, alors que les noeuds 310 et 313 sont connectés à trois conduites.

Il est possible de simuler le fonctionnement du réseau 300 par pas de temps successifs en utilisant un modèle hydraulique. En particulier, il est possible d'intégrer les consommations d'eau (par exemple selon des hypothèses de profil de consommation) aux différents pas de temps, et d'en déduire, à chaque pas de temps, les pressions aux noeuds et vitesses aux arcs du réseau. Le modèle hydraulique choisi peut représenter le profil de consommation moyen du réseau ou des valeurs extrêmes comme le pic de consommation, selon les disponibilités et les souhaits des opérationnels. Il est également possible d'en déduire les évolutions d'autres paramètres physico-chimiques tels que la concentration en chlore, la température, etc... au sein du réseau pour la période de temps simulée.

Dans un ensemble de modes de réalisation de l'invention, des anomalies ou pollutions peuvent être introduites dans différents noeuds du réseau. Ces anomalies peuvent représenter des événements tels qu'une pollution accidentelle ou un acte de malveillance.

Chaque noeud ou arc peut être représenté par un identifiant unique. Le placement d'un capteur peut s'effectuer en affectant à ce capteur l'identifiant unique du noeud ou arc auquel il est placé.

Le secteur 300 est donné à titre d'exemple non-limitatif uniquement. L'invention peut ainsi être appliquée à de nombreux autres réseaux, tels que des réseaux de taille différente (réseaux de la taille d'un quartier, d'une région...), ou des réseaux autres que de distribution d'eau (oléoducs, gazoducs, réseaux de distribution de gaz...).

Dans la suite de la description, l'invention sera illustrée par un cas d'usage consistant à placer des capteurs de manière optimale dans le réseau 300, avec pour objectif de limiter les impacts causés par des dégradations rapides de la qualité de l'eau. Ces dégradations peuvent être limitées en étant détectées rapidement. Celles-ci sont appelées pollutions, peuvent survenir à n'importe quel noeud du réseau de différentes manières :
- Introduction volontaire de substance toxique ;
- Retour d'eau chez un client (particulier ou industriel) ;
- Problème d'infiltration et travaux (remise en eau, absence de désinfection préalable, particules ... ;
- Décrochage important du biofilm du fait d'une vitesse trop importante, causant une augmentation en bactéries et matière organique ;

Les pollutions n'ont pas toutes le même impact selon leur position dans le réseau et le moment auquel elles surviennent. La modélisation hydraulique permet de prendre en compte ces aspects en simulant numériquement les pollutions sur un grand nombre de noeuds du réseau. La modélisation hydraulique permet ainsi une cartographie des impacts causés par les différentes pollutions de manière théorique pour chaque scénario. Cette cartographie des impacts permet de déterminer les combinaisons de noeuds les plus intéressants pour y installer des capteurs et limiter ou détecter au mieux ces impacts. L'invention permet ainsi de couvrir le réseau de manière aussi homogène que possible avec un nombre limité de capteurs, en prenant en compte son comportement hydraulique.

Dans un ensemble de modes de réalisation de l'invention, une pollution est simulée en introduisant dans le modèle numérique, à un pas de temps donné, un polluant avec une concentration donnée. Il est également possible d'introduire dans le modèle numérique une substance non réactive (traceur) avec une concentration en grandeur adimensionnée, par exemple 100. Différentes pollutions peuvent être introduites en différents points selon différents scénarios. Il est également possible d'introduire des anomalies autres que des pollutions (surpressions, croissance bactérienne...). Dans ce cas, on fait l'hypothèse qu'un capteur est capable de détecter une pollution au-dessus d'une certaine concentration de traceur. Ceci est particulièrement efficace pour placer des capteurs tels que le capteur représenté en figure 1, qui sont capables de détecter de nombreux types de pollutions différentes.

Les anomalies peuvent être, selon différents scénarios, injectées en des points critiques, ou points d'intérêts du réseau (réservoirs, usines...), ou encore de manière aléatoire. Le placement de capteurs peut être optimisé pour un ensemble de scénarii correspondant à des apparitions d'anomalies en différents points du réseau.

Dans un ensemble de modes de réalisation de l'invention, des anomalies sont introduites à des noeuds aléatoirement sélectionnés parmi les noeuds ayant strictement plus de 2 canalisations connectées. Ces noeuds peuvent être dénommés les noeuds très connectés, et traduisent bien la structure du réseau en étant connectés à des embranchements principaux et secondaires. Introduire des anomalies à des noeuds aléatoirement sélectionnés parmi les noeuds ayant strictement plus de 2 canalisations connectées permet de réduire significativement le temps de calcul global tout en conservant un grand nombre de scénarios pour avoir une estimation de la distribution des impacts.

Les noeuds auxquels une anomalie peut être introduite peuvent ainsi être aléatoirement sélectionnés parmi une liste des noeuds correspondant au critère choisi (par exemple, noeuds connectés à strictement plus de deux canalisations), listés par leur identifiant unique, par exemple dans la table ci-dessous :

**Tableau 1 - Exemple de liste de noeuds très connectés d'un réseau.**

| |
|---|
| 15968R |
| 21601R |
| 60328R |
| 7174R |
| 7342R |
| 15605R |
| 21390R |
| 16728R |
| 15972R |
| 27659R |
| 23776R |
| 15932R |
| 13956R |
| 20060R |
| 26854R |
| 17958R |
| 20012R |
| 8530R |
| 55822R |
| 17444R |
| 12261R |
| 16070R |
| .... |

Lors de la simulation le polluant introduit, ou la substance constituant le traceur numérique se répand ensuite dans le réseau. La progression de la substance ou du polluant est calculée par pas de temps successifs, via les équations physiques, avec ou sans réaction, par propagation et dilution.

Dans un ensemble de modes de réalisation de l'invention, la capacité des capteurs à détecter une pollution est déterminée à l'aide d'une matrice dite des impacts et d'une matrice dite de détection. La matrice des impacts comprend un scénario par colonne, et un noeud, ou point du réseau, par ligne. Elle donne pour chaque scénario (colonne) et pour l'ensemble des points (lignes) le pas de temps pour lequel la pollution atteint le noeud dans la simulation correspondante. Elle est construite sur la base des temps de parcours après lesquels les quantités de polluants pour chaque noeud sont au-dessus d'une valeur prédéfinie, par exemple 10⁻⁶. Il s'agit ici d'une valeur seuil très faible pour modéliser le fait que des pollutions mêmes très faibles présentent un risque pour les usagers. Un noeud est donc touché même avec une quantité infime du traceur.

Le tableau ci-dessous présente un exemple de matrice des impacts, dans lequel chaque pas de temps représente 10 minutes. Par exemple, la valeur « 36 » dans la cellule en première ligne, 2^{e} colonne signifie que, dans le 2^{e} scénario, la pollution atteint le noeud numéro 1 au bout de 36 x 10 = 360 minutes (6 heures). Ici, les valeurs « 144 » correspondent à un pas de temps maximum de simulation (24h dans cet exemple), auquel la pollution n'a toujours pas atteint le noeud.

**Tableau 2 - Exemple de matrice des impacts**

| | | | | | | | |
|---|---|---|---|---|---|---|---|
| 144 | 36 | 144 | 144 | 144 | 144 | 144 | 144 |
| 144 | 36 | 144 | 144 | 144 | 144 | 144 | 144 |
| 144 | 39 | 144 | 144 | 144 | 144 | 144 | 144 |
| 144 | 39 | 144 | 144 | 144 | 144 | 144 | 144 |
| 144 | 144 | 144 | 144 | 144 | 144 | 144 | 144 |
| 144 | 48 | 144 | 144 | 144 | 144 | 144 | 144 |
| 144 | 84 | 144 | 144 | 144 | 144 | 144 | 144 |
| 144 | 84 | 144 | 144 | 144 | 144 | 144 | 144 |
| 144 | 144 | 144 | 144 | 144 | 144 | 144 | 144 |
| 144 | 84 | 144 | 144 | 144 | 144 | 144 | 144 |
| 144 | 27 | 144 | 144 | 144 | 144 | 144 | 144 |
| 144 | 48 | 144 | 144 | 21 | 144 | 144 | 15 |
| 144 | 54 | 18 | 144 | 144 | 144 | 144 | 144 |
| 144 | 54 | 18 | 144 | 144 | 144 | 144 | 144 |
| 144 | 54 | 18 | 144 | 144 | 144 | 144 | 144 |
| 9 | 45 | 18 | 144 | 144 | 144 | 144 | 144 |
| 9 | 45 | 18 | 144 | 144 | 144 | 144 | 144 |
| 12 | 45 | 21 | 144 | 144 | 144 | 144 | 144 |
| 12 | 45 | 24 | 144 | 144 | 144 | 144 | 144 |
| 144 | 39 | 144 | 6 | 144 | 144 | 144 | 144 |
| 63 | 102 | 78 | 144 | 144 | 144 | 144 | 144 |
| 144 | 69 | 18 | 144 | 144 | 144 | 144 | 144 |
| 144 | 57 | 21 | 144 | 144 | 144 | 144 | 144 |
| 144 | 39 | 144 | 6 | 144 | 144 | 144 | 144 |
| 144 | 66 | 15 | 144 | 144 | 144 | 144 | 144 |

Chaque colonne de la matrice des impacts est propre à chaque scénario, et le temps d'impacts restent constants, quel que soit le placement des capteurs. Dans un ensemble de modes de réalisation de l'invention, la matrice des impacts est ainsi calculée une fois l'ensemble des scénarios simulés, ou a été préalablement calculée et est lue dans un fichier, afin de ne pas recalculer inutilement les impacts de la pollution à chaque placement de capteurs. Dans un mode de réalisation, une fois le calcul de la matrice d'impacts effectué, seul un sous-ensemble des scénarios de la matrice d'impact est utilisé. Par exemple seules les colonnes correspondant à des scénarios de pollutions ayant certaines propriétés peuvent être utilisées : les scénarios occasionnant le plus de noeuds ou de consommation équivalentes impactés, les scénarios occasionnant un pourcentage de noeuds impactés compris entre deux valeurs seuils ... Ceci permet de ne prendre en compte, après avoir simulé les scénarios, que ceux correspondant à un impact que l'on cherche à minimiser.

Une matrice de détections est construite de manière similaire à la matrice d'impacts, mais indique le pas de temps auquel la pollution est détectée, pour un noeud donné. Dans un ensemble de modes de réalisation de l'invention, la pollution est détectée, lorsque la concentration de polluant, ou de traceur, est supérieure à un seuil prédéfini à un noeud équipé d'un capteur. Par exemple, la matrice des détections peut comprendre les pas de temps auxquels une concentration supérieure ou égale 1 a été détectée par un noeud en amont équipé d'un capteur. Ceci permet de simuler le fait que la sensibilité des capteurs est limitée, et que des pollutions peuvent ne pas être détectées, lorsque la concentration en polluant est trop faible, alors même que la pollution a encore un impact.

Selon différents modes de réalisation de l'invention, de nombreuses fonctions objectifs peuvent être établies à partir de la comparaison entre les temps de détection et d'arrivée de la pollution. Par exemple, une fonction objectif peut consister à maximiser un nombre de noeuds pour lesquels une pollution est détectée avant son arrivée. Inversement, une fonction objectif peut consister à minimiser un nombre de points pour lesquels la pollution n'a pas été détectée. Dans ces deux cas, le nombre de points peut être pondéré par un nombre d'usagers, ou une consommation par point. Il peut aussi être divisé par le nombre total de scénarios de pollution ou seulement, pour chaque noeud, par les scénarios ayant réellement impacté chacun des noeuds.

La comparaison des matrices de détections et d'impacts permet donc de construire les listes des noeuds protégés pour un ensemble donné de positions de capteurs et pour chaque scénario d'injection. Dans un ensemble de modes de réalisation de l'invention, la convention utilisée est qu'un noeud est protégé pour un scénario si un capteur détecte la pollution avant qu'elle n'arrive jusqu'à lui. Cette fonction a l'avantage de donner un compromis entre rapidité de détection et étendue de la zone couverte, puisque, d'une part, plus on détecte rapidement et plus on minimise l'impact, et d'autre part, plus on détecte tard et plus potentiellement on est à l'aval du réseau et plus on détecte un nombre de scénarios élevé (i.e lorsqu'une pollution survient au centre du réseau, elle sera par exemple détectée par des noeuds en aval mais pas en amont du réseau ; inversement un capteur situé en amont permettra, lorsqu'il détecte des pollutions en amont, de le faire pour la quasi-totalité des noeuds du réseau). Le coût de chaque scénario non détecté est pondéré par les noeuds et consommations impactées.

Un noeud pourra ainsi être considéré comme protégé si le pas de temps de détection pour ce noeud pour un scénario de pollution donné est strictement inférieur au pas de temps de propagation jusqu'à ce noeud. Dans un ensemble de modes de réalisation de l'invention, ce n'est pas le noeud, mais la consommation de ce noeud qui est considérée comme étant protégée, pour tenir compte des disparités de répartition et favoriser les noeuds avec des consommations importantes. La fonction-objectif utilisée est ainsi la consommation non protégée en moyenne sur l'ensemble des scénarios, ou sur l'ensemble des scénarios impactant de manière différenciée chacun des noeuds, qu'il s'agit de minimiser.

La figure 3 représente un exemple de réseau auquel l'invention peut être appliquée, modélisé sous la forme d'un graphe avec des noeuds et arcs. Cependant, l'invention n'est pas restreinte à cet exemple et l'invention peut être appliquée, par analogie, à d'autres représentations d'un réseau, pour optimiser la capacité des capteurs à détecter des anomalies avant leur arrivée aux différents points du réseau. Par exemple, un maillage en 2D, dans lequel les positions des capteurs sont définies par des coordonnées 2D (x, y) peut être défini. De la même manière, un maillage 3D, dans lequel les positions des capteurs sont définies par des coordonnées 3D (x,y,z) peut être utilisé.

La figure 4 représente un exemple de détection d'anomalie dans un réseau de distribution de fluide selon un ensemble de modes de réalisation de l'invention.

L'exemple de la figure 4 est basé sur une portion 400 d'un réseau de distribution d'eau.

La portion 400 de réseau comprend 9 noeuds 410, 411, 412, 413, 414, 415, 416, 417, 418 reliés par des arcs formant 3 branches. Le sens d'écoulement de l'eau va du noeud 410 vers les noeuds 411, 412, 413 successivement, puis du noeud 413 vers les noeuds 414, 415, et vers les noeuds 416, 417, 418 respectivement. Cette portion de réseau 400 est équipée d'un unique capteur au noeud 417.

Dans un scénario de simulation, une pollution est injectée 420, à un pas de temps, au noeud 411. Cette pollution se propage progressivement aux noeuds 412, 413, puis aux noeuds 414, 415 d'une part, et 416, 417, 418 d'autre part.

Cette pollution n'est donc, en l'absence de capteur, pas détectée pour les noeuds 411, 412, 413, 414, 415, 416. Elle est en revanche détectée par le capteur pour le noeud 417. Elle est également détectée pour le noeud 418. Il est en effet possible, dès que la pollution est détectée pour le noeud 417, de déduire que cette pollution affectera les noeuds en aval tel que le noeud 418, et d'en déduire les actions correctrices appropriées. Les outils de suivi peuvent également permettre d'analyser si la pollution risque de se répandre dans d'autres zones du réseau.

Dans un ensemble de modes de mise en oeuvre de l'invention, le score de performance pour un placement de capteur est déterminé en évaluant les noeuds pour lesquels la pollution est détectée avant son arrivée, et ceux pour lesquels la pollution est détectée après son arrivée, ou pas détectée du tout. Ceci peut s'effectuer par exemple en notant pour chaque noeud, le pas de temps auquel la pollution arrive, et le pas de temps auquel la pollution est détectée, et en comparant les deux. Par exemple, des matrices d'impacts et de détection, telles que définies en référence à la figure 3, peuvent être utilisées.

Dans l'exemple de la figure 4 :
- la pollution n'est pas détectée pour les noeuds 411, 412, 413, 414, 415, 416 ;
- la pollution est détectée après son arrivée pour le noeud 417. En effet, par nature, au moment où les mesures indiquant une pollution sont effectuées et traitées, la pollution est déjà arrivée au noeud 417 ;
- la pollution est détectée avant son arrivée pour le noeud 418. En effet, lorsque la pollution est détectée sur la base des mesures du capteur au noeud 417, il est possible de déduire que la pollution atteindra le noeud 418, avant même son arrivée, avec éventuellement un temps de latence.

Le placement des capteurs permet une détection plus ou moins efficace des pollutions. Dans l'exemple de la figure 4, un placement du capteur au noeud 412 aurait par exemple permis de détecter la pollution avant son arrivée, pour les noeuds 413, 414, 415, 416, 417, 418.

L'utilisation d'algorithmes génétiques pour le placement des capteurs permet de définir, de manière itérative, un placement de capteurs permettant d'augmenter la probabilité de détecter une anomalie avant son arrivée pour un nombre de points du réseau aussi élevé que possible, quel que soit le lieu d'apparition de l'anomalie.

De manière générale, l'application d'algorithmes génétiques au placement de capteurs associé à ce type de détection d'anomalies a pour effet de positionner les capteurs de manière à ce qu'ils détectent les pollutions en moyenne avant que les noeuds à l'aval ne soient touchés : c'est-à-dire que l'ensemble des noeuds étant touchés par le front de pollution plus tard que le capteur seront protégés. Si un capteur perçoit la pollution très en amont, des branches entières du réseau peuvent être protégées.

D'autres fonctions objectifs peuvent être utilisées selon différents modes de réalisation de l'invention. Par exemple, les fonctions suivantes peuvent être utilisées, seules ou en combinaison :
- la minimisation du linéaire moyen de réseau contaminé (une canalisation est considérée comme contaminée si elle située entre deux noeuds contaminés) ;
- la minimisation d'un sous-ensemble de noeuds contaminés parmi l'ensemble des noeuds (le sous-ensemble peut typiquement contenir des noeuds « critiques » ou « sensibles » tels que des noeuds correspondant à des usagers sensibles comme les hôpitaux, les écoles ...) ;
- la minimisation du temps de détection moyen des pollutions ;
- la minimisation du pourcentage de scénarios de pollution non détectés. Cette fonction donne des résultats assez différents des autres car le temps après lequel la pollution est introduite et donc son impact, n'importent pas.

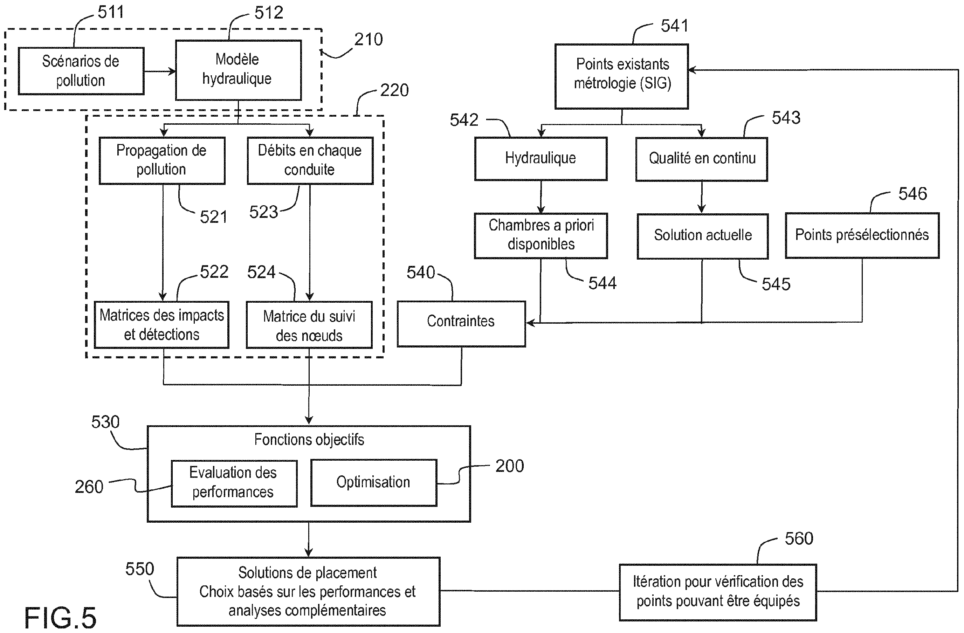

La figure 5 représente un exemple de définition de contraintes d'optimisation selon un ensemble de modes de mise en oeuvre de l'invention.

Une méthode selon l'invention peut être paramétrée selon de nombreuses contraintes et objectifs. Par exemple, des paramètres d'entrée 210 peuvent être définis. Ces paramètres d'entrée peuvent notamment comprendre des scénarii de pollution 511, et un modèle hydraulique 512. Ces paramètres d'entrée permettent de simuler 220 le fonctionnement du réseau dans chaque scénario.

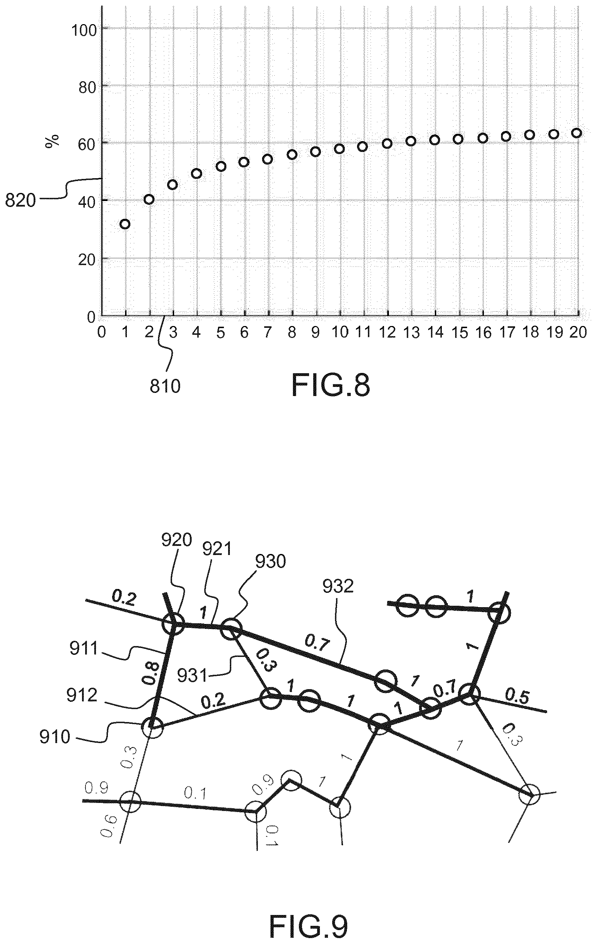

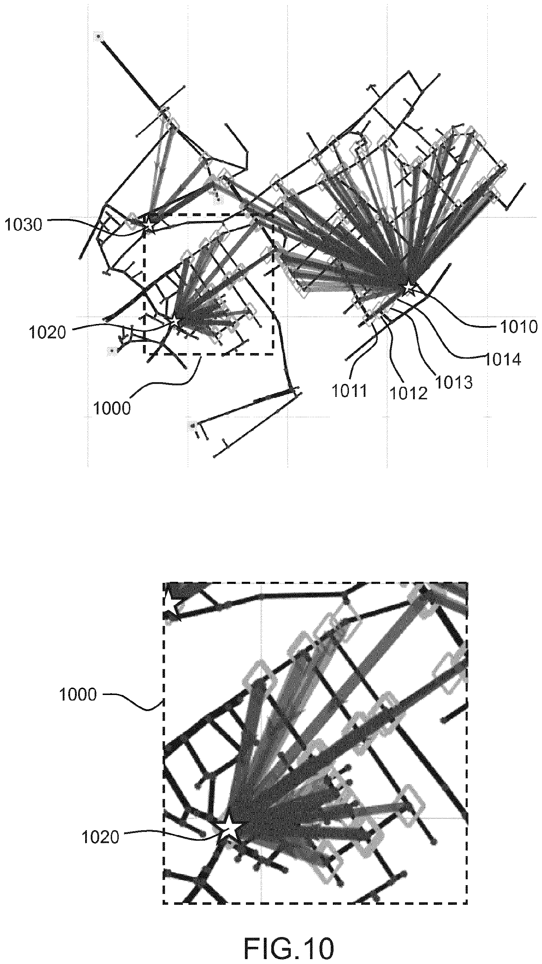

Notamment, les paramètres d'entrée 210 permettent de déterminer 521 la propagation de la pollution, afin d'obtenir une matrice des impacts 522 représentant le temps d'arrivée de la pollution pour chaque noeud. Ils permettent également de déterminer 523 les débits dans chaque conduites, afin d'obtenir une matrice 524 de suivi des noeuds indiquant quels noeuds sont liés les uns aux autres, et de déterminer si un noeud permet de suivre la qualité de l'eau dans des noeuds amont. La construction de telles matrices est notamment décrite en référence aux figures 9 et 10.

La méthode selon l'invention peut également optimiser la position des capteurs, uniquement sur un sous-ensemble 540 de points du réseau. Ces points sont sélectionnés parmi en ensemble de points existants 541.

La méthode selon l'invention peut intégrer les équipements déjà prévus déployés dans le réseau, tels que :
- les capteurs hydrauliques déjà déployés 542 ;
- les capteurs de qualité déjà déployés 543 ;
- les chambres de mesures disponibles 544 : des chambres de mesures, comprenant déjà des capteurs, peuvent être utilisées pour déployer des capteurs qualité à moindre coût ;
- la solution actuelle 545, comprenant les capteurs de qualité déjà déployés ;
- les noeuds présélectionnés 546, comprenant les noeuds que l'on souhaite équiper quoi qu'il en soit (sites sensibles, réservoirs, entrées de réseau...).

Une méthode selon l'invention peut également être paramétrée avec une ou plusieurs fonctions objectif 530 indiquant l'objectif suivi par le placement des capteurs. Ces fonctions objectifs permettent de calculer 250 des scores de performances, lors de l'exécution 200 de la méthode de placement de capteurs selon l'invention.

La solution de placement optimale obtenue peut ainsi être analysée 550 par un opérateur, pour s'assurer qu'elle est satisfaisante. Dans le cas où cette solution serait sous-optimale, par exemple si un point de placement n'a pas été retenu car il ne faisait pas partie du sous-ensemble de points autorisés pour l'optimisation, ce problème peut être détecté par l'opérateur, qui peut ainsi vérifier les contraintes de placement des capteurs, et les modifier si nécessaire 560, avant de procéder à une nouvelle itération de placement.

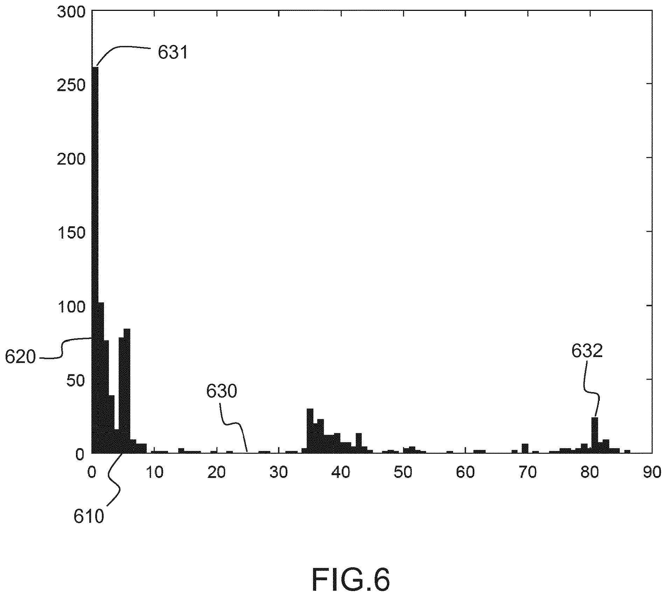

La figure 6 représente un exemple d'histogramme représentant les pourcentages de noeuds d'un réseau de distribution d'eau impactés par une pollution selon 940 scénarios de tests pour le placement de capteurs sur le secteur 300.

La figure 6 représente :
- sur l'axe des abscisses 610, les pourcentages de noeuds touchés pour les différents scénarios ;
- sur l'axe des ordonnées 620, le nombre de scénarios correspondant à chaque pourcentage de noeuds impactés.

La figure 6 montre ainsi que le pourcentage de noeuds impactés par les pollutions est très variable selon les scénarios : dans plus de 250 scénarios, entre 0 et 1% des noeuds sont impactés 632, alors que, dans une vingtaine de scénarios, plus de 80% des noeuds sont impactés, et qu'un nombre quasi nul de scénarios impacte entre 20 et 30% des noeuds 630.

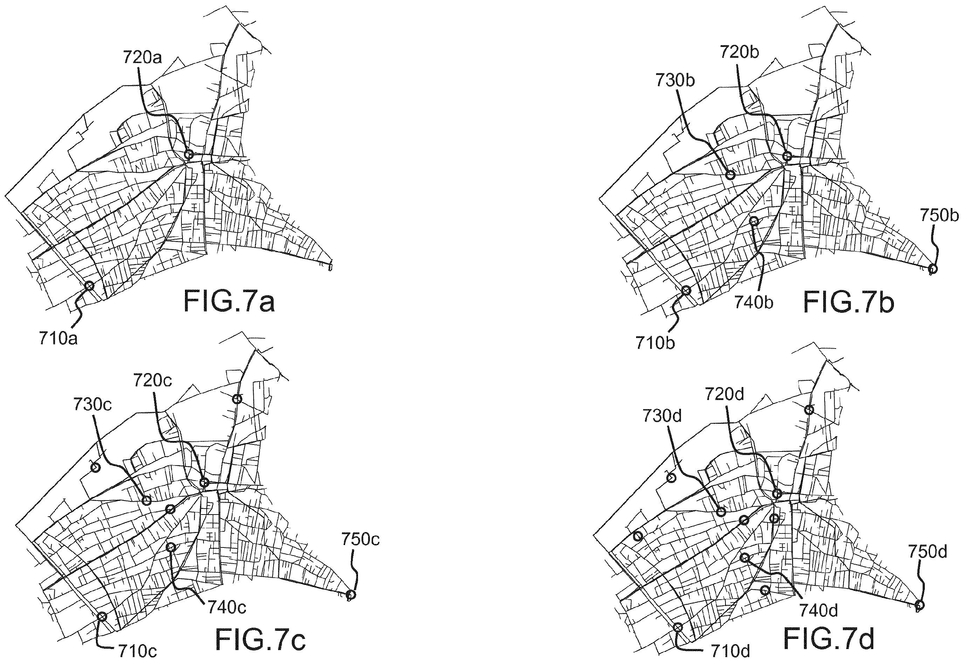

Les figures 7a, 7b, 7c et 7d représentent quatre exemples de placement de 2, 5, 8 et 11 capteurs respectivement dans un réseau de distribution d'eau par une méthode de placement de capteurs dans un mode de réalisation de l'invention visant uniquement la détection de pollution.

Dans cet exemple, une méthode d'optimisation de placement de capteurs selon l'invention a été exécutée avec les paramètres ci-dessous.

Les noeuds sur lesquels les capteurs pouvaient être placés dans cet exemple répondaient à différents critères :
- ils sont connectés à strictement plus de 2 arcs ;
- ils ont un minimum de détection de scénarios de 5. Ceci peut être réalisé, en déterminant, pour chaque scénario, les noeuds auxquels la pollution peut être détectée. On peut ensuite, inversement, déterminer, pour chaque noeud, le nombre de scénarios de pollutions qu'il sera capable de détecter, et présélectionner les noeuds qui sont capables de détecter la pollution pour un nombre de scénarios supérieur à un seuil prédéfini, par exemple 5 ;
- les noeuds sur lesquels un capteur est déjà déployé, ou peu favorables au déploiement d'un capteur (contrainte terrain nécessitant un investissement trop élevé, contraintes sur d'éventuels travaux de voirie, circulation importante...) sont éliminés.

La limitation de l'optimisation à certains noeuds permet d'éliminer les noeuds auxquels le placement de capteur est impossible et/ou de simplifier le problème et l'exécution de la méthode selon l'invention, tout en basant l'optimisation sur les noeuds a priori les plus favorables. Cependant, ces contraintes sont données à titre d'exemple non limitatif uniquement, et d'autres contraintes peuvent être utilisées. Par exemple, le nombre minimum de détection de scénarios peut dépendre du nombre de capteurs à tester, afin de s'adapter à la complexité du problème.

L'optimisation est alors réalisée, successivement pour un nombre de capteurs allant de 1 à 20.

La pluralité d'ensembles candidats de positions est initialement obtenue, en sélectionnant de manière aléatoire les capteurs parmi les noeuds présélectionnées, puis plusieurs itérations d'optimisation par algorithme génétique sont effectuées.

A chaque itération, un score est calculé pour chaque ensemble candidat, en fonction d'un pourcentage de la consommation non couverte par les mesures capteurs (i.e les noeuds pour lesquels une pollution n'est pas détectée sont identifiés, pondérés par leur consommation relative et rapportés au nombre total de noeuds, pour obtenir un pourcentage de la consommation pour lequel une pollution n'a pas été détectée. Plus ce pourcentage est faible, meilleur est l'ensemble candidat).

L'algorithme génétique est ensuite appliqué pour modifier la pluralité d'ensembles candidats à chaque itération, en fonction des scores, selon les règles suivantes :
- les 5% d'ensembles candidats les meilleurs sont conservés (sélection des élites) ;
- 80% d'ensembles candidats intermédiaires sont croisés entre eux, et/ou avec les « élites » (meilleurs candidats) pour générer de nouveaux ensembles ;
- les 15% d'ensembles candidats ayant obtenus les moins bons scores mutent, c'est-à-dire qu'ils sont modifiés aléatoirement.

Cette application de la méthode 200 selon l'invention permet d'obtenir un placement optimal pour chaque nombre de capteurs. Une fois la méthode effectuée pour un nombre N de capteurs, la solution optimale utilisant N capteurs est utilisée comme base pour l'optimisation de N+1 capteurs.

On obtient ainsi un placement optimal pour chacun des nombres de capteurs entre 1 et 20.

Ces exemples montrent que, de manière générale, la méthode selon l'invention produit un placement relativement homogène des capteurs sur le réseau, permettant ainsi de détecter des anomalies survenant en tout point. Un avantage de l'invention est de ne conserver, lors de l'ajout d'un capteur, les positions des capteurs précédemment placés, que si cette position reste optimale. C'est le cas dans les figures 7a à 7d :
- les deux capteurs 710a, 720a sont conservés lorsque le nombre de capteurs augmente 710b, 720b, 710c, 720c, 710d, 720d ;
- les trois capteurs additionnels 730c, 740b, 750b de la solution à 5 capteurs sont conservés lorsque le nombre de capteurs passe à 8 (730c, 740c, 750c) ou 11 (730d, 740d, 750d).
Cependant, contrairement à d'autres algorithmes tels que les algorithmes dits « gloutons », dans l'invention, si l'ajout d'un nouveau capteur rend la position des capteurs précédemment placés non-optimale, cette position sera modifiée.

La figure 8 représente l'évolution d'un pourcentage de détection d'anomalies par des capteurs dans un réseau de distribution d'eau placés par une méthode selon l'invention, en fonction du nombre de capteurs.

L'axe des abscisses 810 représente le nombre de capteurs placés, entre 1 et 20, dans l'exemple précédemment évoqué. L'axe des ordonnées 820 représente le pourcentage de noeuds considérés comme couverts, c'est-à-dire le pourcentage de noeuds pour lesquels une pollution est détectée avant son arrivée.

La figure 8 montre que l'augmentation du nombre de capteur permet initialement des gains de protection importants. Le gain de protection marginal associé à l'ajout d'un nouveau capteur devient de moins en moins important au fur et à mesure que le nombre de capteurs déployés augmente. L'invention permet ainsi de choisir un nombre de capteur optimal, soit en fonction d'un taux de couverture cible, soit en fonction d'une optimisation pourcentage de couverture / coût de déploiement.

Les optimisations présentées en référence aux figures 7a à 7d, et 8 sont données à titre d'exemple non limitatif uniquement. L'invention n'est pas restreinte à ces exemples, et la méthode selon l'invention peut optimiser le placement de capteurs selon de nombreuses contraintes différentes.

Par exemple, dans certains secteurs, l'optimisation peut se faire uniquement sur un sous-ensemble des points, afin d'exploiter au mieux les infrastructures existantes.

Des secteurs ayant des réservoirs de grandes tailles peuvent être optimisés en introduisant un point de mesure à l'aval des réservoirs afin de suivre les pollutions qui pourraient spécifiquement les toucher. Ces points sont considérés comme fixés et inclus dans les populations de combinaisons de capteurs testées. Ils ont pour effet de détecter des pollutions au niveau des réservoirs. Si les réservoirs ne sont pas utilisés comme points d'intrusion dans les scénarios testés, l'apport de ces points dans les performances est nul.

Un secteur sur lequel se trouve un hôpital peut générer la contrainte complémentaire d'équiper le point d'entrée de celui-ci, qui est en fait l'un des noeuds de consommation du modèle hydraulique. Ceci est pris en compte pour l'optimisation comme forçage de la solution. Le point de l'hôpital est ainsi présent dans l'ensemble des combinaisons évaluées dans le processus d'optimisation. Il participe aux performances de détection en détectant les scénarios de pollution situés à l'amont de l'hôpital et pouvant toucher d'autres points.

Un autre secteur avec plusieurs installations sensibles (i.e installations militaires, industrielles...) peut être optimisé en pondérant les noeuds du modèle correspondant à ces sites, afin qu'ils soient privilégiés dans le processus d'optimisation. La fonction objectif permet ainsi de placer les capteurs en favorisant la protection des sites sensibles contre les pollutions, voire en ne protégeant que ceux-ci en les pondérant à 1 et tous les autres à 0.

Le fait de pouvoir choisir d'optimiser du réseau dans son ensemble, ou de manière différenciée selon les secteurs permet ainsi plus de souplesse et d'adaptation aux contraintes terrain et à l'expertise des professionnels de la distribution de l'eau potable. L'optimisation par secteurs de tous les secteurs permet également de diminuer les temps de calculs par rapport à l'optimisation globale.

La méthode selon l'invention peut ainsi s'adapter à des contextes opérationnels très différents, et permet un placement de capteurs optimal, pour des objectifs très différents. La méthode selon l'invention peut être configurée par l'utilisateur, en fonction de sa connaissance des réseaux sur lesquels elle est appliquée.

La figure 9 représente un exemple de répartition des débits dans un réseau de distribution d'eau.

Une méthode selon l'invention peut être utilisée pour optimiser le placement de capteurs pour la détection d'anomalies ou de pollution. Elle peut être également utilisée pour d'autres fins, comme optimiser un suivi de qualité de l'eau. A cet effet, le score affecté à chaque ensemble candidat de positions de capteurs peut être calculé à partir d'une fonction consistant à déterminer le nombre de noeuds par lequel a transité l'eau au moment de la mesure. Ce type de suivi, dit suivi aval, peut reposer sur la génération d'une matrice de fractionnement de débits. Pour l'ensemble des noeuds, les pourcentages de débit transitant dans chacune des conduites adjacentes à l'aval est calculé à différents pas de temps.

La figure 9 fournit un exemple de fractionnement de débits. La figure 9 indique les sens d'écoulement de l'eau, et les fractions de débit allant dans chaque conduite. L'écoulement dans le réseau d'effectue généralement, dans cet exemple, de la gauche vers la droite. Les nombres indiqués sur chaque canalisation indique la fraction du débit qui va d'un noeud vers un autre. Par exemple :
- pour 1 unité, le débit entrant au noeud 910 est fractionné à 0,8 (80%) dans la conduite 911, et à 0,2 (20%) dans la conduite 912 ;
- pour 1 unité, le débit entrant au noeud 920 est fractionné à 1 (100%) dans la conduite 921 ;
- pour 1 unité, le débit entrant au noeud 930 est fractionné à 0,3 (30%) dans la conduite 931, et à 0,7 (70%) dans la conduite 932.

Cette relation au niveau des noeuds directement adjacents est ensuite propagée sur l'ensemble du réseau. Dans un ensemble de modes de réalisation de l'invention, on définit une relation de suivi aval pour un couple de noeuds si l'un des deux reçoit une fraction majoritaire du débit ayant transité par l'autre (>50%). Les liaisons représentées en gras montrent les noeuds considérés comme liés entre eux. Par exemple, le noeud 910 est considéré comme lié au noeud 920, et le noeud 920 au lieu 930. Si le seuil de 50% de débit fournit une bonne indication de la liaison entre deux noeuds, d'autres seuils (40%, 60%...) peuvent également être utilisés.

Il est ainsi possible, pour chacun des noeuds du graphe, de déterminer le nombre de noeuds amont auxquels il est lié, c'est-à-dire le nombre de noeuds par lequel l'eau reçu aura transité. Le noeuds peuvent également être affectés de la population, ou de la consommation associée. Ainsi, on peut déduire, pour chaque noeud, quelle population, ou consommation d'eau sera couverte par un suivi de qualtié effectué à ce noeud.

La figure 10 représente un exemple de suivi de la qualité de l'eau par des capteurs situés sur des noeuds en aval d'un réseau de distribution d'eau.

La figure 10 montre comment les noeuds entourés de losanges sont considérés comme suivis par les noeuds entourés d'une étoile. Les flux sont symboliquement représentés, faisant ressortir à ce pas de temps la structure et le comportement hydraulique du réseau.

La figure 10 représente le secteur 300, dont la portion 1000 est représentée agrandie en bas d'image. Dans l'exemple de la figure 10, les noeuds ont été liés de manière itérative, lorsque plus de 50% du débit entrant dans un noeud transite vers le noeud suivant. Par exemple, le noeud 1011 est lié au noeud 1012, lui-même lié au noeud 1013, etc... jusqu'au noeud 1010. Le noeud 1011 est donc lié 1014 au noeud 1010. La figure 10 représente tous les noeuds liés à un autre avec un losange, et les liaisons entre les noeuds par des traits gris épais, tels que le trait 1014. La figure 10 montre qu'une grande partie des noeuds du réseau convergent soit vers le noeud aval 1010, soit vers le noeud aval 1020. Cette représentation permet donc de mieux appréhender la structure des débits et du comportement hydraulique du réseau, à un pas de temps donné.

Des capteurs placés aux noeuds 1010 et 1020 permettent ainsi de vérifier la qualité de l'eau sur une grande partie du réseau.

Une méthode selon l'invention permet de placer des capteurs pour suivre de manière optimale la qualité de l'eau, en calculant le score de performance à l'aide d'une fonction objectif définissant le nombre de noeuds auxquels un noeud est lié, c'est-à-dire un nombre de noeuds pour lesquels un noeud peut effectuer un suivi aval. Une telle fonction objectif permet de placer les capteurs de manière à pouvoir suivre des qualités d'eau différentes provenant des différentes sources et surtout des différentes zones de mélange de ces sources. De manière générale, les capteurs placés à l'aide de cette fonction objectif peuvent également détecter des pollutions ayant lieu en aval du réseau.

Cette fonction objectif peut être utilisée seule, ou en combinaison avec une fonction objectif concernant la détection d'anomalie ou de pollution. La présence de capteurs pour le suivi aval peut ainsi compléter les capteurs placés avec la fonction objectif de détection de pollution. Cette combinaison est également bien adaptée au découpage du réseau en sous-lots d'eau correspondant à une source ou un mélange de source.

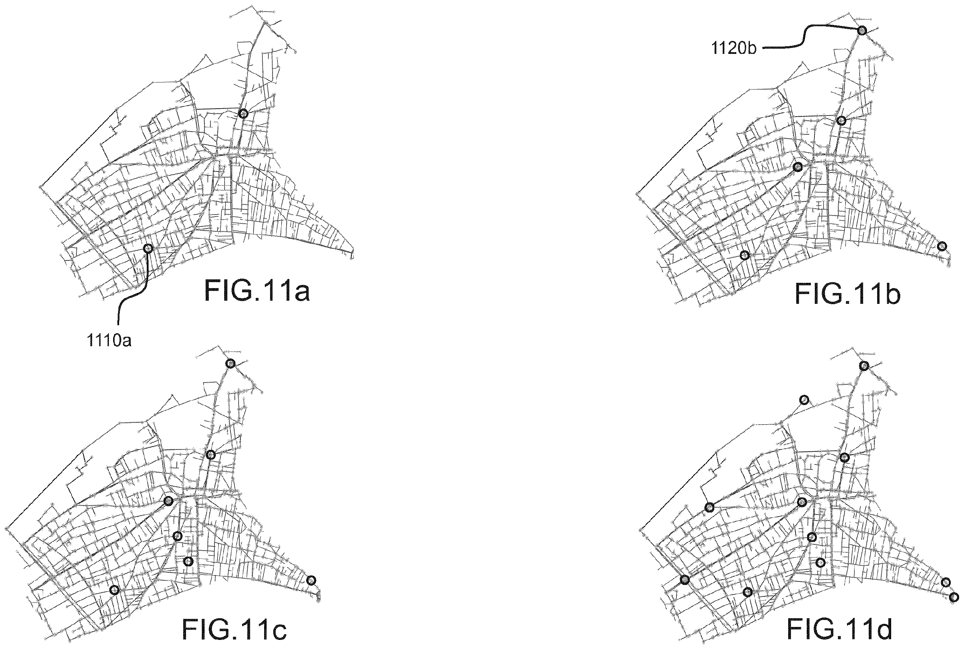

Les figures 11a, 11b, 11c et 11d représentent quatre exemples de placement de 2, 5, 8 et 11 capteurs respectivement dans un réseau de distribution d'eau par une méthode de placement de capteurs dans un mode de réalisation de l'invention visant à la fois la détection de pollution et le suivi de la qualité de l'eau.

Le placement des capteurs est effectué sur le même principe que pour l'exemple des figures 7a à 7d, exception faite des points ci-dessous.

Comme indiqué ci-dessus, afin de simplifier le problème tout en obtenant de bonnes performances, les capteurs peuvent être placés uniquement dans un sous-ensemble des noeuds du réseau. Comme expliqué en référence aux figures 9 et 10, les noeuds permettant un placement optimal des capteurs pour le suivi aval de la pollution sont des noeuds liés à un grand nombre de noeuds du réseau situés en amont. Le sous-ensemble des noeuds sur lesquels le placement de capteurs est possible peut donc comprendre les noeuds connectés, selon la règle définie en référence à la figure 9 à un nombre de noeuds en amont supérieur ou égal à un seuil prédéfini. La consommation par noeud peut également être considérée, et le sous-ensemble de noeuds sur lequel l'optimisation est effectuée peut comprendre les noeuds connectés à un nombre de noeuds en amont correspondant à une consommation supérieure à un seuil prédéfini.

Pour optimiser de manière conjointe le placement de capteurs, pour la détection d'anomalies et le suivi aval, il est pertinent de présélectionner des noeuds formant de bons candidats pour la détection d'anomalie, et des noeuds formant de bons candidats pour le suivi aval de la qualité de l'eau. Par exemple, le sous-ensemble de noeuds sur lequel l'optimisation est effectuée peut comprendre des noeuds connectés à un grand nombre de noeuds en amont (formant de bons candidats pour le suivi aval), et des noeuds connectés à un nombre d'arcs supérieur à un seuil prédéfini (formant de bons candidats pour la détection d'anomalies).

Lors de l'optimisation, le score affecté à chacun des ensembles candidats de positions est calculé à partir d'une fonction objectif combinée, égale à la moyenne d'une fonction objectif de détection d'anomalie et d'une fonction objectif de suivi de qualité de l'eau, afin de favoriser les ensembles candidats de positions permettant à la fois une bonne détection d'anomalies, et un bon suivi de la qualité de l'eau.

L'algorithme génétique est ensuite appliqué pour modifier la pluralité d'ensembles candidats à chaque itération, en fonction des scores, selon les règles suivantes :
- les 5% d'ensembles candidats les meilleurs sont conservés (sélection des élites) ;
- 80% d'ensembles candidats intermédiaires sont croisés entre eux et/ou les noeuds « élites » (meilleurs candidats conservés) pour générer de nouveaux ensembles ;
- les 15% d'ensembles candidats ayant obtenus les moins bons scores mutent, c'est-à-dire qu'ils sont modifiés aléatoirement.

Cette application de la méthode 200 selon l'invention permet d'obtenir un placement optimal pour chaque nombre de capteurs. Une fois la méthode effectuée pour un nombre N de capteurs, la solution optimale utilisant N capteurs est utilisée comme base pour l'optimisation de N+1 capteurs.

On obtient ainsi un placement optimal pour chacun des nombres de capteurs entre 1 et 20.

Les placements des noeuds sont représentés, pour 2, 5, 8 et 11 noeuds respectivement, par de cercles tels que le cercle 1110a. Pour un même nombre de capteurs, le placement est ici différent de celui réalisé pour les figures 7a à 7d. Par exemple, dans la solution à 5 capteurs, le capteur 1120b est placé dans une zone où aucun capteur n'était situé dans la figure 7b (solution à 5 capteurs pour la détection de pollution uniquement). Ce capteur 1120b permet un suivi de la qualité de l'eau sur toute la partie nord du réseau, et a donc permis à la solution à 5 capteurs présentée en figure 11b d'obtenir un bon score pour la combinaison « détection d'anomalies + suivi de qualité de l'eau ».

Ces exemples démontrent la capacité de l'invention à fournir un placement optimal de capteur en fonction d'un objectif défini par l'utilisateur. L'invention est donc applicable à un grand nombre de cas d'usages, et permet d'obtenir un placement optimal de capteurs selon un grand nombre de critères différents.

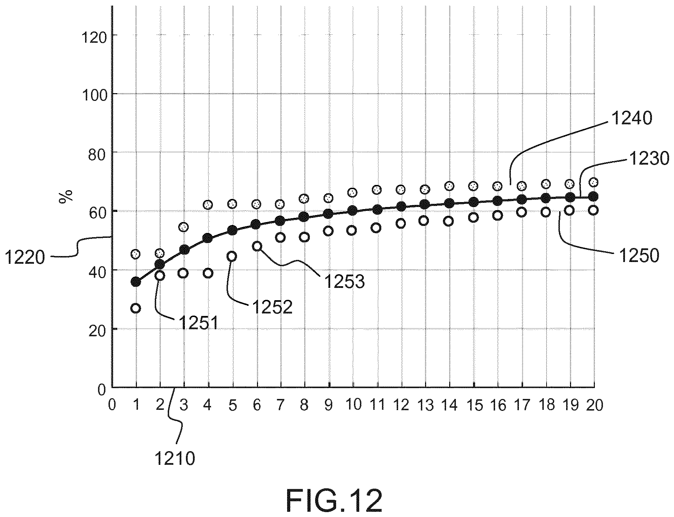

La figure 12 représente l'évolution d'un score combiné de détection de pollution et de suivi de la qualité de l'eau en fonction d'un nombre de capteurs placés par une méthode selon l'invention.

Les courbes de la figure 12 sont créées sur le même principe que la courbe de la figure 8 : l'axe des abscisses 1210 représente le nombre de capteurs placés, et l'axe vertical 1220 représente le score de performance de la solution optimale, exprimé en pourcentage, et la courbe 1230 l'évolution du score en fonction du nombre de capteurs, dans l'exemple des figures 11a à 11d.

Comme indiqué ci-dessus, dans l'exemple d'optimisation combiné des figures 11a à 11d, le score de performance est calculé comme la moyenne d'un score de suivi de la qualité de l'eau, et d'un score de détection d'anomalies. La courbe supérieure 1240 représente le score de suivi de la qualité de l'eau, et la courbe inférieure 1250 le score de détection d'anomalies. Lors de l'ajout d'un nouveau capteur, l'augmentation du score global peut se faire par augmentation du score de suivi de la qualité de l'eau et/ou du score de détection d'anomalies. Par exemple, les ajouts des 3^{e} et 4^{e} capteurs ont ici principalement amélioré le score de suivi de la qualité de l'eau 1241, 1242, alors que les ajouts des 2^{e}, 5^{e} et 6^{e} capteurs ont principalement amélioré le score de détection d'anomalies 1251, 1252, 1253.

Cet exemple démontre la capacité de l'invention à disposer les capteurs de manière optimisée selon une combinaison d'objectifs, en améliorant l'un ou l'autre des objectifs pour obtenir le meilleur score global.

Les exemples ci-dessus démontrent la capacité de l'invention à optimiser le placement de capteurs selon différents critères cibles. Ils ne sont cependant donnés qu'à titre d'exemple et ne limitent en aucun cas la portée de l'invention, définie dans les revendications ci-dessous.

## Revendications

1. Méthode de placement d'une pluralité de capteurs d'un ou plusieurs paramètres physico-chimiques d'un fluide, ladite méthode comprenant :
- une détermination d'un ensemble de positions dans un réseau de transport du fluide, ladite détermination comprenant les étapes d'une méthode (200) mise en oeuvre par ordinateur comprenant les étapes suivantes :
- l'obtention (220), pour au moins un ensemble de paramètres d'entrée (210), de valeurs simulées de grandeurs physico-chimiques en un ensemble de points d'un réseau de transport d'un fluide pour un ensemble de pas de temps ;
- l'obtention (230) d'une pluralité d'ensembles candidats (240) de positions dans le réseau d'une pluralité de capteurs d'un ou plusieurs paramètres physico-chimiques du fluide ;
- de manière itérative, jusqu'à la validation (260) d'un critère d'arrêt :
∘ l'obtention (250), pour chaque ensemble candidat de positions, d'un score de performance au moins à partir des valeurs simulées de grandeurs physico-chimiques aux positions de la pluralité de capteurs ;
∘ la modification (270) de la pluralité d'ensembles candidats des positions de capteurs, ladite modification comprenant au moins une opération choisie parmi :
▪ la conservation (271) d'au moins un ensemble candidat de positions ayant un score le plus favorable ;
▪ l'ajout (272) d'au moins un ensemble candidat des positions de capteurs défini par une combinaison des positions d'au moins deux ensembles candidats ;
▪ l'ajout (273) d'au moins un ensemble candidat des positions de capteurs défini par une modification d'une position d'un capteur dans un ensemble candidat ;
- la sélection (290) de l'ensemble candidat de positions ayant le score le plus favorable.
- un placement desdits capteurs dans ledit ensemble de positions.

2. Méthode selon la revendication 1, dans laquelle :
- le réseau est modélisé sous forme d'un graphe ;
- chaque noeud ou arc du graphe est identifié par un identifiant unique ;
- la position d'un capteur est définie par un identifiant d'un noeud ou arc du graphe.

3. Méthode selon l'une des revendications 1 ou 2, dans laquelle au moins un des ensembles de paramètres d'entrée (210) comprend l'introduction d'une anomalie dans au moins un point du réseau à au moins un pas de temps.

4. Méthode selon la revendication 3, dans laquelle le score de performance est calculé en fonction de la capacité des capteurs placés dans l'ensemble candidat de positions à détecter l'au moins une anomalie en déterminant un pas de temps d'arrivée de l'au moins une anomalie en un ensemble de points du réseau, et au moins une fonction objectif choisie parmi :
- un nombre de points de l'ensemble, pour lesquels l'anomalie est détectée avant son arrivée ;
- un nombre de points de l'ensemble, pour lesquels l'anomalie est détectée avant son arrivée, pondérés par un nombre d'usagers ou une consommation par point ;
- un nombre de points pour lesquels l'anomalie n'a pas été détectée ;
- un nombre de points pour lesquels l'anomalie n'a pas été détectée, pondérés par un nombre d'usagers ou une consommation par point.

5. Méthode selon l'une des revendications 1 à 4, dans lequel le score de performance est calculé à partir d'au moins une caractéristique, choisie parmi une capacité des capteurs d'un ensemble candidat à évaluer un indicateur de qualité du fluide dans le réseau, et un coût de déploiement des capteurs.

6. Méthode selon l'une des revendications 1 à 5, dans laquelle au moins un capteur a une position prédéfinie.

7. Méthode selon l'une des revendications 1 à 6, dans laquelle les points auxquels les capteurs sont placés sont restreints à un sous-ensemble des points du réseau.

8. Méthode selon l'une des revendications 1 à 7, dans laquelle la définition de la pluralité d'ensembles candidats de positions de la pluralité de capteurs comprend la définition de positions à des points d'intérêt du réseau.

9. Méthode selon l'une des revendications 1 à 8, dépendant de la revendication 2, dans laquelle la définition de la pluralité d'ensembles candidats de positions de la pluralité de capteurs comprend la définition de positions à des noeuds connectés à un nombre d'arcs supérieur ou égal à un seuil prédéfini.

10. Méthode selon l'une des revendications 1 à 9, dans lequel le critère d'arrêt comprend une ou plusieurs conditions choisies parmi :
- un nombre d'itérations maximal ;
- une comparaison du score le plus favorable parmi l'itération courante et au moins une itération précédente, et la validation du critère d'arrêt si une différence entre le score le plus favorable à l'itération courante et à l'au moins une itération précédente est inférieure ou égale à un seuil prédéfini.

11. Méthode selon l'une des revendications 1 à 10, dans laquelle :
- la suppression d'au moins un ensemble candidat de positions n'ayant pas le score le plus favorable comprend la suppression de tous les ensembles candidats sauf un nombre prédéfini, ou un ratio prédéfini des ensembles candidats ayant le score le plus favorable ;
- l'ajout (272) d'au moins un ensemble candidat des positions de capteurs défini par une combinaison des positions d'au moins deux d'ensembles candidats, et l'ajout (273) d'au moins un ensemble candidat des positions de capteurs défini par une modification d'une position d'un capteur dans un ensemble candidat ajoutent un nombre d'ensembles candidats égal au nombre d'ensembles candidats supprimés.

12. Méthode selon l'une des revendications 1 à 11, comprenant la définition, la modification de manière itérative, et la sélection d'ensembles candidats de capteurs pour une pluralité de nombres prédéfinis de capteurs respectivement.

13. Méthode selon la revendication 12, dans laquelle l'obtention (230) d'une pluralité d'ensembles candidats de positions de la pluralité de capteurs pour un nombre entier (n) de capteurs est basée sur l'ensemble candidat de position ayant le score le plus favorable pour un nombre entier (n-m) de capteurs, avec 1 ≤ m < n.

14. Méthode selon l'une des revendications 12 ou 13, comprenant la sélection d'un des ensembles candidats ayant le score le plus favorable pour la pluralité de nombres prédéfinis de capteurs, en fonctions des scores et coûts de déploiement de chacun desdits ensembles candidats.

15. Produit programme d'ordinateur comprenant des instructions de code de programme qui, lorsque le programme est exécuté par un ordinateur, conduisent celui-ci à mettre en oeuvre la méthode selon l'une des revendications 1 à 14.

16. Dispositif apte à déterminer un ensemble de positions, dans un réseau de transport d'un fluide, d'une pluralité de capteurs d'un ou plusieurs paramètres physico-chimiques du fluide, ledit dispositif comprenant un processeur configuré pour exécuter la méthode selon l'une des revendications 1 à 13.

## Patentansprüche

1. Verfahren zur Platzierung einer Vielzahl von Sensoren für einen oder mehrere physikalisch-chemische Parameter einer Flüssigkeit, wobei das Verfahren Folgendes umfasst:
- eine Bestimmung eines Satzes von Positionen in einem Netzwerk zum Transport der Flüssigkeit, wobei die Bestimmung die Schritte eines computerimplementierten Verfahrens (200) umfasst, das die folgenden Schritte umfasst:
- Erhalten (220), für mindestens einen Satz von Eingangsparametern (210), von simulierten Werten physikalisch-chemischer Größen an einem Satz von Punkten eines Transportnetzes einer Flüssigkeit für einen Satz von Zeitschritten;
- Erhalten (230) einer Vielzahl von Kandidatensätzen (240) von Positionen in dem Netzwerk einer Vielzahl von Sensoren für einen oder mehrere physikalisch-chemische Parameter der Flüssigkeit;
- iterativ bis zur Bestätigung (260) eines Abbruchkriteriums:
○ Erhalten (250) einer Leistungsbewertung für jeden Kandidatensatz von Positionen zumindest aus den simulierten Werten physikalisch-chemischer Größen an den Positionen der Vielzahl von Sensoren;
○ Änderung (270) der mehreren Kandidatensätze der Sensorpositionen, wobei die Änderung mindestens einen Vorgang umfasst, der ausgewählt ist aus:
■ Beibehalten (271) von mindestens einem Kandidatensatz von Positionen mit der günstigsten Punktzahl;
■ Hinzufügen (272) von mindestens einem Kandidatensatz von Sensorpositionen, der durch eine Kombination der Positionen von mindestens zwei Kandidatensätzen definiert ist;
■ Hinzufügen (273) von mindestens einem Kandidatensatz von Sensorpositionen, der durch eine Änderung einer Sensorposition in einem Kandidatensatz definiert ist;
- Auswahl (290) des Kandidatensatzes von Positionen mit der günstigsten Punktzahl.
- eine Platzierung der Sensoren in dem Satz von Positionen

2. Verfahren nach Anspruch 1, wobei:
- das Netzwerk als Graph modelliert wird;
- jeder Knoten oder Bogen des Graphen durch eine eindeutige Kennung identifiziert wird;
- die Position eines Sensors durch eine Kennung eines Knotens oder Bogens des Graphen definiert wird.

3. Verfahren nach einem der Ansprüche 1 oder 2, wobei mindestens einer der Sätze von Eingangsparametern (210) die Einführung einer Anomalie in mindestens einem Punkt des Netzwerks bei mindestens einem Zeitschritt umfasst.

4. Verfahren nach Anspruch 3, bei dem die Leistungsbewertung auf der Grundlage der Fähigkeit der Sensoren berechnet wird, die in dem Kandidatensatz von Positionen angeordnet sind, um die mindestens eine Anomalie zu erkennen, indem ein Zeitschritt der Ankunft der mindestens einen Anomalie an einer Menge von Punkten des Netzwerks bestimmt wird, und mindestens eine Zielfunktion, die ausgewählt ist aus:
- einer Anzahl von Punkten in der Menge, bei denen die Anomalie vor ihrer Ankunft erkannt wird;
- einer Anzahl von Punkten in der Menge, bei denen die Anomalie vor ihrem Eintreffen erkannt wird, gewichtet mit einer Anzahl von Nutzern oder einem Verbrauch pro Punkt;
- einer Anzahl von Punkten, bei denen die Anomalie nicht erkannt wurde;
- einer Anzahl von Punkten, bei denen die Anomalie nicht erkannt wurde, gewichtet mit einer Anzahl von Nutzern oder einem Verbrauch pro Punkt.

5. Verfahren nach einem der Ansprüche 1 bis 4, wobei die Leistungsbewertung aus mindestens einem Merkmal berechnet wird, das aus einer Fähigkeit der Sensoren eines Kandidatensatzes, einen Indikator für die Qualität der Flüssigkeit im Netzwerk zu bewerten, und den Kosten für den Einsatz der Sensoren ausgewählt ist.

6. Verfahren nach einem der Ansprüche 1 bis 5, bei dem mindestens ein Sensor eine vordefinierte Position hat.

7. Verfahren nach einem der Ansprüche 1 bis 6, wobei die Punkte, an denen die Sensoren platziert werden, auf eine Teilmenge der Punkte des Netzwerks beschränkt sind.

8. Verfahren nach einem der Ansprüche 1 bis 7, wobei das Definieren der mehreren Kandidatensätze von Positionen der mehreren Sensoren das Definieren von Positionen an interessanten Punkten im Netzwerk umfasst.

9. Verfahren nach einem der Ansprüche 1 bis 8, abhängig von Anspruch 2, wobei die Definition der mehreren Kandidatensätze von Positionen der Vielfalt von Sensoren das Definieren von Positionen an Knoten umfasst, die mit einer Anzahl von Bögen verbunden sind, die größer oder gleich einem vordefinierten Schwellenwert ist.

10. Verfahren nach einem der Ansprüche 1 bis 9, wobei das Abbruchkriterium eine oder mehrere Bedingungen umfasst, die ausgewählt sind aus:
- einer maximale Anzahl von Iterationen;
- einem Vergleich des günstigsten Ergebnisses zwischen der aktuellen Iteration und mindestens einer vorhergehenden Iteration und die Validierung des Abbruchkriteriums, wenn ein Unterschied zwischen dem günstigsten Ergebnis der aktuellen Iteration und der mindestens einen vorhergehenden Iteration kleiner oder gleich einem vordefinierten Schwellenwert ist.

11. Verfahren nach einem der Ansprüche 1 bis 10, wobei:
- das Entfernen von mindestens einem Kandidatensatz von Positionen, die nicht die günstigste Punktzahl haben, das Entfernen aller Kandidatensätze bis auf eine vordefinierte Anzahl oder ein vordefiniertes Verhältnis der Kandidatensätze mit der günstigsten Punktzahl umfasst;
- das Hinzufügen (272) von mindestens einem Kandidatensatz von Sensorpositionen, der durch eine Kombination der Positionen von mindestens zwei der Kandidatensätze definiert ist, und das Hinzufügen (273) von mindestens einem Kandidatensatz von Sensorpositionen, der durch eine Änderung einer Position eines Sensors in einem Kandidatensatz definiert ist, eine Anzahl von Kandidatensätzen hinzufügt, die gleich der Anzahl von gelöschten Kandidatensätzen ist.

12. Verfahren nach einem der Ansprüche 1 bis 11, umfassend das Definieren, iterative Ändern und Auswählen von Sensorkandidatensätzen für eine Vielzahl jeweils vordefinierter Anzahlen von Sensoren.

13. Verfahren nach Anspruch 12, wobei das Erhalten (230) einer Vielzahl von Positionskandidatensätzen der Vielzahl von Sensoren für eine ganze Zahl (n) von Sensoren auf dem Positionskandidatensatz mit der günstigsten Punktzahl für eine ganze Zahl (n-m) von Sensoren basiert, wobei 1 ≤ m < n gilt.

14. Verfahren nach einem der Ansprüche 12 oder 13, umfassend die Auswahl eines der Kandidatensätze mit der günstigsten Punktzahl für die Vielzahl von vordefinierten Sensorzahlen, basierend auf den Punktzahlen und Kosten für den Einsatz jedes dieser Kandidatensätze.

15. Computerprogrammprodukt mit Programmcodeanweisungen, die, wenn das Programm von einem Computer ausgeführt wird, diesen dazu veranlassen, das Verfahren nach einem der Ansprüche 1 bis 14 auszuführen.

16. Vorrichtung, die geeignet ist, in einem Transportnetz für eine Flüssigkeit einen Satz von Positionen einer Vielzahl von Sensoren für einen oder mehrere physikalisch-chemische Parameter der Flüssigkeit zu bestimmen, wobei die Vorrichtung einen Prozessor umfasst, der so konfiguriert ist, dass er das Verfahren nach einem der Ansprüche 1 bis 13 ausführt.

## Claims

1. A method of placing several sensors of one or more physico-chemical parameters of a fluid, said method comprising:
- a determination of a set of positions in a fluid transport network, said determination comprising the steps of a computer-implemented method (200) comprising the following steps:
- obtaining (220), for at least one set of input parameters (210), simulated values of physico-chemical quantities at a set of points of a fluid transport network for a set of time steps;
- obtaining (230) a plurality of candidate sets (240) of positions in the network of a plurality of sensors for one or more physico-chemical parameters of the fluid;
- iteratively, until a stop criterion is validated (260):
○ obtaining (250), for each candidate set of positions, a performance score at least from the simulated values of physico-chemical quantities at the positions of the plurality of sensors;
○ modifying (270) the plurality of candidate sets of sensor positions, said modifying comprising at least one operation selected from:
■ retaining (271) at least one candidate set of positions with a most favourable score;
■ adding (272) at least one candidate set of sensor positions defined by a combination of the positions of at least two candidate sets;
■ adding (273) at least one candidate set of sensor positions defined by modifying a position of a sensor in a candidate set;
- selection (290) of the candidate set of positions with the most favourable score.
- placing said sensors in said set of positions

2. A method according to claim 1, wherein:
- the network is modelled as a graph;
- each node or arc in the graph is identified by a unique identifier;
- the position of a sensor is defined by an identifier of a node or arc in the graph.

3. A method according to one of claims 1 or 2, in which at least one of the sets of input parameters (210) comprises the introduction of an anomaly in at least one point of the network at at least one time step.

4. A method according to claim 3, wherein the performance score is calculated as a function of the ability of sensors placed in the candidate set of positions to detect the at least one anomaly by determining a time step of arrival of the at least one anomaly at a set of points in the network, and at least one objective function selected from:
- a number of points in the set for which the anomaly is detected before it arrives;
- a number of points in the set, for which the anomaly is detected before it arrives, weighted by a number of users or consumption per point;
- a number of points for which the anomaly was not detected;
- a number of points for which the anomaly was not detected, weighted by the number of users or consumption per point.

5. A method according to one of claims 1 to 4, in which the performance score is calculated from at least one characteristic, chosen from a capacity of the sensors of a candidate set to assess an indicator of the quality of the fluid in the network, and a cost of deploying the sensors.

6. A method according to one of claims 1 to 5, in which at least one sensor has a predefined position.

7. The method of any of claims 1 to 6, wherein the points at which the sensors are placed are restricted to a subset of the points in the network.

8. The method of any one of claims 1 to 7, wherein defining the plurality of candidate sets of positions of the plurality of sensors comprises defining positions at points of interest in the network.

9. A method according to any one of claims 1 to 8, dependent on claim 2, wherein defining the plurality of candidate sets of positions of the plurality of sensors comprises defining positions at nodes connected to a number of arcs greater than or equal to a predefined threshold.

10. The method of any one of claims 1 to 9, wherein the stopping criterion comprises one or more conditions selected from:
- a maximum number of iterations;
- a comparison of the most favourable score between the current iteration and at least one previous iteration, and validation of the stopping criterion if a difference between the most favourable score at the current iteration and at least one previous iteration is less than or equal to a predefined threshold.

11. The method according to one of claims 1 to 10, wherein:
- removing at least one candidate set of positions that do not have the most favourable score comprises removing all but a predefined number, or a predefined ratio, of candidate sets with the most favourable score;
- the addition (272) of at least one candidate set of sensor positions defined by a combination of the positions of at least two of the candidate sets, and the addition (273) of at least one candidate set of sensor positions defined by a modification of a position of a sensor in a candidate set add a number of candidate sets equal to the number of deleted candidate sets.

12. A method according to any of claims 1 to 11, comprising defining, iteratively modifying, and selecting candidate sets of sensors for a plurality of predefined numbers of sensors respectively.

13. The method of claim 12, wherein obtaining (230) a plurality of position candidate sets of the plurality of sensors for an integer number (n) of sensors is based on the position candidate set with the most favourable score for an integer number (n-m) of sensors, with 1 ≤ m < n.

14. A method according to any of claims 12 or 13, comprising selecting one of the candidate sets having the most favourable score for the plurality of predefined numbers of sensors, as a function of the scores and deployment costs of each of said candidate sets.

15. A computer programme product comprising program code instructions which, when the programme is executed by a computer, cause the computer to implement the method of any one of claims 1 to 14.

16. A device capable of determining a set of positions, in a fluid transport network, of a plurality of sensors of one or more physico-chemical parameters of the fluid, said device comprising a processor configured to perform the method according to one of claims 1 to 13.
